Europäisches Patentamt

European Patent Office

Office européen des brevets

(11) Publication number: **0 162 067 B1**

# EUROPEAN PATENT SPECIFICATION

(45) Date of publication of patent specification: **15.07.92**

(51) Int. Cl.5: **C12Q 1/68**, C12P 21/00, C12P 21/02, C12N 15/10, C12N 15/12, C12N 1/20, C12N 1/16, C12N 1/18, C12N 1/00, C07H 15/12, C12N 15/85

(21) Application number: **84904025.8**

(22) Date of filing: **12.10.84**

(86) International application number: **PCT/US84/01641**

(87) International publication number: **WO 85/01961 (09.05.85 85/11)**

Divisional application 85202121 filed on 19.12.85.

(54) **PRODUCTION OF FACTOR VIII AND RELATED PRODUCTS.**

(30) Priority: **28.10.83 US 546650**
**24.08.84 US 644036**

(43) Date of publication of application:
**27.11.85 Bulletin 85/48**

(45) Publication of the grant of the patent:
**15.07.92 Bulletin 92/29**

(84) Designated Contracting States:
**AT BE CH DE FR GB LI LU NL SE**

(56) References cited:
EP-A- 0 083 483      EP-A- 0 130 756
EP-A- 0 150 735      EP-A- 0 157 556
EP-A- 0 160 457      WO-A-84/04541

NATURE, vol. 306, no. 5943, 8th December 1983, page 528 MacMillan Journals Ltd, Chesham, Bucks, GB; J. MADDOX: "Factor VIII cloning"

(73) Proprietor: **GENETICS INSTITUTE, INC.**
**87 Cambridge Park Drive**
**Cambridge, Massachusetts 02140(US)**

(72) Inventor: **TOOLE, John, J., Jr.**
**27 Lakeville Road**
**Jamaica Plain, MA 02130(US)**

(74) Representative: **Weinhold, Peter, Dr. et al**
**Patentanwälte Dipl.-Ing. G. Dannenberg Dr.**
**P. Weinhold Dr. D. Gudel Dipl.-Ing. S. Schubert Dr. P. Barz Siegfriedstrasse 8**
**W-8000 München 40(DE)**

NATURE, vol. 303, 9th June 1983, pages 474, 475 Macmillan Journals Ltd, GB; A.L. BLOOM: "Haemophilia: benefits of cloning genes for clotting facotrs"

GENET. BIOTECHNOL. BACILLI, PROC. INT. CONF. 2nd, held in Stanford 6-8 July 1983 (Publ. 1984) ed. by Ganesan, A.T. et al. Academic: Orlando, Fla. US; J.A. WELLS et als.: "Cloning and sequencing of a region controlling efficient expression of subtilising from Bacillus amyloliquefaciens"

GENE, vol. 20, 1987, pages 387-396 Elsevier Biomedical Press, NL; J.H. KINNAIRD et al.: "Cloning of the am (glutamate dehydrogenase) gene of Neurospora crassa through the use of a synthetic DNA probe"

CELL, vol. 14, July 1978, pages 673-680 D.L. MONTGOMERY et al.: "Identification and isolation of the yeast cytochrome c gene"

PROC.NATL.ACAD.SCI. USA, vol. 78, no. 11, November 1981, pages 6613-6617 US; S.V. SUGGS et al.: "Use of synthetic obligonucleotides as hybridization probes: isolation of cloned cDNA sequences for human beta2-microglobulin"

Choo et al., Nature, Vol. 299, 178-180, September 1982

Kurachi et al. Proc. Natl. Acad. Sci. USA, Vol. 79, pp. 6461-6464, November 1982

Jaye et al., Nucleic Acids Research, Vol. 11, No. 8, pp. 2325-2335, 1983

Knutson et al., Blood, Vol. 59, pp. 615-624, March 1982

Maniatis et al., Molecular Cloning, A Laboratory Manual, Cold Spring Harbor Laboratory, pp. 224-228, 1982

Tuddenham et al., J. Lab. Clin. Med. Vol. 93, No. 1, pp. 40-53, January 1979

Tuddenham et al., J. Lab. Clin. Med. Vol. 93, No. 1, pp. 40-53, January

**Description**

Background of the Invention

This invention relates to the preparation of recombinant deoxyribonucleic acid (DNA) which codes for cellular production of human factor VIII:C, and of DNA which codes for porcine factor VIII:C, to methods of obtaining DNA molecules which code for factor VIII:C, and to expression of human and porcine factor VIII:C utilizing such DNA, as well as to novel compounds, including deoxyribonucleotides and ribonucleotides utilized in obtaining such clones and in achieving expression of human factor VIII:C. This invention also relates to human AHF and its production by recombinant DNA techniques.

Factor VIII:C is a blood plasma protein that is defective or absent in Hemophilia A disease. This disease is a hereditary bleeding disorder affecting approximately one in 20,000 males. Factor VIII:C has also been known or referred to as factor VIII, the antihemophilic factor (AHF), antihemophilic globulin (AHG), hemophilic factor A, platelet cofactor, thromboplastinogen, and thrombocytolysin. It is referred to as "Factor VIII:C", to indicate that it is the compound which affects clotting activity. As used herein, "factor VIII:C" and "AHF" are synonymous.

Although the isolation of AHF from blood plasma has been described in the literature, the precise structure of AHF has not previously been identified, due in part to the unavailability of sufficient quantities of pure material, and the proteolytic nature of many contaminants and purification agents. While some quantities of impure AHF have been available as a concentrated preparation processed from fresh-frozen human plasma, the extremely low concentration of AHF in human plasma and the high cost of obtaining and processing human plasma make the cost of this material prohibitive for any extensive treatment of hemophilia.

The present invention makes it possible to produce human AHF using recombinant DNA techniques.

AHF, like other proteins, is comprised of some twenty different amino acids arranged in a specific array. By using gene manipulation techniques, a method has been developed which enables production of AHF by identifying and cloning the gene which codes for the human AHF protein, cloning that gene, incorporating that gene into a recombinant DNA vector, transfoming a suitable host with the vector which includes that gene, expressing the human AHF gene in such host, and recovering the human AHF produced thereby. Similarly, the present invention makes it possible to produce porcine AHF by recombinant DNA techniques, as well as providing products and methods related to such porcine AHF production.

Recently developed techniques have made it possible to employ microorganisms, capable of rapid and abundant growth, for the synthesis of commercially useful proteins and peptides, regardless of their source in nature. These techniques make it possible to genetically endow a suitable microorganism with the ability to synthesize a protein or peptide normally made by another organism. The technique makes use of fundamental relationships which exist in all living organisms between the genetic material, usually DNA, and the proteins synthesized by the organism. This relationship is such that production of the amino acid sequence of the protein is coded for by a series of trinucleotide sequences of the DNA. There are one or more trinucleotide sequence groups (called codons) which specifically code for the production of each of the twenty amino acids most commonly occurring in proteins. The specific relationship between each given trinucleotide sequence and the corresponding amino acid for which it codes constitutes the genetic code. As a consequence, the amino acid sequence of every protein or peptide is reflected by a corresponding nucleotide sequence, according to a well understood relationship. Furthermore, this sequence of nucleotides can, in principle be translated by any living organism. For a discussion of the genetic code, see J. D. Watson, Molecular Biology of the Gene, (W. A. Benjamin, Inc., 1977) particularly at 347-77; C. F. Norton, Microbiology (Addison, Wesley 1981), and U. S. Patent No. 4,363,877.

The twenty amino acids from which proteins are made, are phenylalanine (hereinafter sometimes referred to as "Phe" or "F"), leucine ("Leu", "L"), isoleucine ("Ile", "I"), methionine ("Met", "M"), valine ("Val", "V"), serine ("Ser", "S"), proline ("Pro", "P"), threonine ("Thr", "T"), alanine ("Ala", "A"), tyrosine ("Tyr", "Y"), histidine ("His", "H"), glutamine ("Gln", "Q"), asparagine ("Asp", "N"), glutamic acid ("Glu", "E"), cysteine ("Cys", "C"), tryptophane ("Trp", "W"), arginine ("Arg", "R") and glycine ("Gly", "G"). The amino acids coded for by the various combinations of trinucleotides which may be contained in a given codon may be seen in Table 1:

## TABLE 1

### The Genetic Code

| First Position | Second Position | | | | Third Position |
|---|---|---|---|---|---|
| | T | C | A | G | |
| T | Phe | Ser | Tyr | Cys | T |
| | Phe | Ser | Tyr | Cys | C |
| | Leu | Ser | Stop* | Stop* | A |
| | Leu | Ser | Stop* | Trp | G |
| C | Leu | Pro | His | Arg | T |
| | Leu | Pro | His | Arg | C |
| | Leu | Pro | Gln | Arg | A |
| | Leu | Pro | Gln | Arg | G |
| A | Ile | Thr | Asp | Ser | T |
| | Ile | Thr | Asp | Ser | C |
| | Ile | Thr | Lys | Arg | A |
| | Met | Thr | Lys | Arg | G |
| G | Val | Ala | Asp | Gly | T |
| | Val | Ala | Asp | Gly | C |
| | Val | Ala | Glu | Gly | A |
| | Val | Ala | Glu | Gly | G |

*The "Stop" or termination codon terminates the expression of the protein.

Knowing the deoxyribonucleotide sequence of the gene or DNA sequence which codes for a particular protein allows the exact description of that protein's amino acid sequence. However, the converse is not true; while methionine is coded for by only one codon, the other amino acids can be coded for by up to six codons (e.g. serine), as is apparent from Table 1. Thus there is considerable ambiguity in predicting the nucleotide sequence from the amino acid sequence.

In sum, prior to the present invention, very little was known about the structure of AHF, and, despite substantial work over many years, those skilled in this art were unable to determine the structure of AHF, or of its gene, or provide any procedure by which AHF could be produced in substantially pure form in substantial quantities.

The method described herein by which the gene for human AHF is cloned and expressed includes the following steps:

(1) Purification of porcine AHF;

(2) Determination of the amino acid sequence of porcine AHF;

(3) Formation of oligonucleotide probes, and use of those probes to identify and/or isolate at least a fragment of the gene which codes for procine AHF;

(4) Use of the porcine AHF gene fragment to identify and isolate human genetic material which codes for human AHF;

(5) Using the previously described AHF DNA fragments to determine the site of synthesis of AHF from among the various mammalian tissues;

(6) Producing cDNA segments which code for human and porcine AHF, using messenger RNA obtained from the tissue identification in step 5;

(7) Constructing full length human and porcine cDNA clones from the cDNA segments produced in step 6, e.g. by ligating together cDNA segments which were cut by the same restriction enzymes;

(8) Forming DNA expression vectors which are capable of directing the synthesis of AHF;

(9) Transforming a suitable host with the expression vectors bearing the full length cDNA for human or porcine AHF;

(10) Expressing human or porcine AHF in the host; and

(11) Recovering the expressed AHF.

In the course of this work, a new technique of screening a genomic DNA library has been developed utilizing oligonucleotide probes based on the amino acid sequences contained in the AHF molecule.

The invention includes the above methods, along with the various nucleotides, vectors, and other products made in connection therewith.

Brief Summary of the Drawings

Figure 1 is a depiction of the amino acid sequence (A) determined for the amino terminal sequence of the 69,000 dalton thrombin cleavage product described in Example 1. The first residue was not identifiable. This sequence is compared with sequence (B) deduced from the nucleotide sequence of the porcine AHF exon described in Example 3, and with the human AHF exon sequence(C) described in Example 4.

Figure 2 illustrates the amino acid sequence, shown in single letter code, of bovine thrombin digestion fragments of (A) the amino terminus and (B) a 40 Kd thrombin cleavage product of the 166 Kd porcine AHF fragment isolated by Fass et al., infra.

Figure 3 illustrates the design of an oligonucleotide probe for the identification and isolation of at least a portion of the porcine gene which codes for AHF.

Figure 4 illustrates the DNA sequence for a Sma I DNA fragment (34-S1) which contains a porcine AHF exon, derived from bacteriophage PB34 described in Example 3.

Figure 5 is a representation of the DNA sequence of the Hae III insert 34-H1 bearing the exon for porcine AHF, as described in Example 3. This sequence is included within the longer sequence shown in Figure 4 and corresponds to nucleotides 250-615 of the sequence in Figure 4.

Figure 6 is a representation of the DNA sequence, showing a portion of the exon for human AHF, as described in Example 4.

Figure 7 illustrates the DNA nucleotide sequence (shown in one strand only) which contains the entire sequence coding for human AHF, along with the deduced amino acid sequence for human AHF.

Detailed Description of the invention

The following definitions are supplied in order to facilitate the understanding of this case. To the extent that the definitions vary from meanings circulating within the art, the definitions below are to control.

Amplification means the process by which cells produce gene repeats within their chromosomal DNA.

Cotransformation means the process of transforming a cell with more than one exogenous gene foreign to the cell, one of which confers a selectable phenotype on the cell.

Downstream means the direction going towards the 3' end of a nucleotide sequence.

An enhancer is a nucleotide sequence that can potentiate the transcription of genes independent of the identity of the gene, the position of the sequence in relation to the gene, or the orientation of the sequence.

A gene is a deoxyribonucleotide sequence coding for a given mature protein. For the purposes herein, a gene shall not include untranslated flanking regions such as RNA transcription initiation signals, polyadenylation addition sites, promoters or enhancers.

A selection gene is a gene that confers a phenotype on cells which express the gene as a detectable protein.

A selection agent is a condition or stance that enables one to detect the expression of a selection gene.

Phenotype means the observable properties of a cell as expressed by the cellular genotype.

Genotype means the genetic information contained within a cell as opposed to its expression, which is observed as the phenotype.

Ligation is the process of forming a phosphodiester bond between the 5' and 3' ends of two DNA strands. This may be accomplished by several well known enzymatic techniques, including blunt end ligation by T4 ligase.

Orientation refers to the order of nucleotides in a DNA sequence. An inverted orientation of a DNA sequence is one in which the 5' to 3' order of the sequence relation to another sequence is reversed when compared to a point of reference in the DNA from which the sequence was obtained. Such points of reference can include the direction of transcription of other specified DNA sequences in the source DNA or the origin of replication of replicable vectors containing the sequence.

5

Transcription means the synthesis of RNA from a DNA template.

Transformation means changing a cell's genotype by the cellular uptake of exogenous DNA. Transformations may be detected in some cases by an alteration in cell phenotype. Transformed cells are called transformants. Pre-transformation cells are referred to as parental cells.

Translation means the synthesis of a polypeptide from messenger RNA (mRNA).

The present invention permits, for the first time, the identification and isolation of porcine Factor VIII:C gene by recombinant DNA techniques. It also permits, for the first time, the isolation and identification of the gene which encodes human factor VIII:C by recombinant DNA techniques, by taking advantage of the homology between porcine AHF DNA and human AHF DNA in order to locate and isolate the human gene for AHF. This route to cDNA clones for producing AHF via homology with the porcine AHF gene avoids the tedious time consuming and expensive need to purify human AHF, which is highly expensive and essentially unavailable.

Porcine AHF is first highly purified, most preferably by a monoclonal antibody purification technique such as that disclosed by David Fass et al, "Monoclonal Antibodies to Porcine AHF Coagulant and Their Use in the Isolation of Active Coagulant Protein", Blood 59: 594-600 (1982), and Knutsen et al., "Porcine Factor VIII:C prepared by affinity Interaction with Von Willebrand Factor and Heterologous Antibodies," Blood, 59: 615-24 (1982). Porcine factor VIII:C polypeptides are bound to anti-VIII:C monoclonal antibodies which are immobilized on a suitable affinity chromatography column. Two large molecular weight polypeptides, having molecular sizes of about 166 and 130 Kd, are eluted with ethylenediamine tetraacetic acid. Another protein segment having a molecular weight of about 76 Kd, is then eluted from the column, utilizing about 50% ethylene glycol. The partial amino acid sequences of these polypeptides, and/or of fragments of these polypeptides obtained in a known manner e.g. from enzymatic digestion of the proteins, using bovine thrombin or other suitable agents to break up the proteins, are then determined by known methods of analysis. Based on the amino acid sequence of these materials, oligonucleotide probes are synthesized, at least some of which will hybridize with DNA segments which code for the corresponding segment of AHF. These oligonucleotides are then used to screen for segments of the gene which code for porcine AHF.

Once a portion of the porcine gene for AHF is obtained, that recombinant material is used to screen a human genomic DNA library to locate and isolate the gene which codes for human AHF. In this procedure, it is established that there are substantial similarities between human AHF and porcine AHF, and advantage is taken of those similarities to isolate and identify the human factor VIII:C gene or gene segments.

The similarities in the porcine and human proteins are attributable to corresponding similarities in the DNA sequences which code for the amino acid sequences. The genetic materials coding for the AHF proteins in humans and pigs are identical at a high percentage of positions, and thus edit hybridization when subjected to the procedure of, for example, Benton and Davis, "Screening λ gt Recombinant Clones by Hybridization to Single Plaques In Situ," Science, 196:180 (1977).

The cloned segments of the gene for human AHF are then used to identify a source for AHF mRNA from among various human tissues. Similarly, one or more of the cloned segments for porcine AHF are used to screen potential tissue sources for porcine mRNA. This step is accomplished by conventional procedures involving RNA extraction, gel electrophoresis, transfer to nitrocellulose sheets, and hybridization to radiolabelled cloned DNA as described for example in Maniatis, et al., Molecular Cloning, A Laboratory Manual, (Cold Spring Harbor Laboratory, 1982). Once this tissue source is identified, cDNA libraries are prepared (in either plasmid or preferably bacteriophage lambda vectors, both of which are generally available) and screened for AHF cDNA clones as described below. The process of cDNA library screening is repeated until a set of cDNA clones is obtained which, by DNA sequence determination, is shown to comprise the entire DNA gene sequence encoding AHF.

Once the full length human or porcine AHF cDNA clone is obtained, known and appropriate means are utilized to express the AHF protein, e.g. insertion into an appropriate vector, and transfection into an appropriate host, selection of transformed cells (transformants), and culture these transformants, to express AHF activity.

Host-vector systems for the expression of AHF may be procaryotic, but the complexity of AHF makes the preferred expression system eucaryotic, preferably (at least for biologically-active AHF having clotting activity) a mammalian one. This is easily accomplished by eucaryotic (usually mammalian or vertebrate cells) transformation with a suitable AHF vector. Eucaryotic transformation is in general a well-known process, and may be accomplished by a variety of standard methods. These include the use of protoplast fusion, DNA microinjection, chromosome transfection, lytic and nonlytic viral vectors (For example, Mulligan et al., "Nature" (London) 277:108-114 (1979), cell-cell fusion (Fournier et al., "Proc. Nat. Acad. Sci."74:319-323 (1977), lipid structures (U.S. patent 4,394,448) and cellular endocytosis of DNA precipitates (Bachetti et al., "Proc. Nat. Acad. Sci."74:1590-1594 (1977). Other eucaryotic cells, such as yeasts or insect cells, may also be used to advantage.

Transformation which is mediated by lytic viral vectors is efficient but is disadvantageous for a number of reasons: The maximum size of transfected DNA is limited by the geometry of viral capsid packing, the exogenous genes are frequently deleted during viral replication, there is a requirement for helper virus or specialized hosts, host cells must be permissive, and the hosts are killed in the course of the viral infection.

Nonlytic transformations are based on the transcription and translation of virus vectors which have been incorporated into a cell line as a stable episome. These systems generally require unique cell line and suffer from a number of disadvantages. See "Trends in Biochemical Sciences", June 1983, pp. 209-212.

On the other hand, other transformation methods in which extrachromosomal DNA is taken up into the chromosomes of host cells have been characterized by low frequencies of transformation and poor expression levels. These initial difficulties were ameliorated by transformation with genes which inheritably confer selectable phenotypes on the small subpopulation of cells that are in fact transformed (selection genes). The entire population of transformed cells can be grown under conditions favoring cells having acquired the phenotype, thus making it possible to locate transformed cells conveniently. Thereafter, transformants can be screened for the capability to more intensely express the phenotype. This is accomplished by changing a selection agent in such a way as to detect higher expression.

Selection genes fall into three categories: Detectably amplified selection genes, dominant selection genes, and detectably amplified dominant selection genes.

Detectably amplified selection genes are those in which amplification can be detected by exposing host cells to changes in the selection agent. Detectably amplified genes which are not dominant acting generally require a parental cell line which is genotypically deficient in the selection gene. Examples include the genes for hydroxymethylglutamyl CoA reductase (Sinensky, "Biochem. Biophys. Res. Commun. " 78:863 (1977), ribonucleotide reductase (Meuth et al. "Cell:3:367 (1943), aspartate transcarbamylase; (Kemp et al. "Cell" 9:541 (1976), adenylate deaminase (DeBatisse et al., "Mol and Cell Biol." 2(11):1346-1353 (1982) mouse dihydrofolate reductase (DHFR) and, with a defective promoter, mouse thymidine kinase (TK).

Dominant selection genes are those which are expressed in transformants regardless of the genotype of the parental cell. Most dominant selection genes are not detectably amplified because the phenotype is so highly effective in dealing with the selection agent that it is difficult to discriminate among cell lines that have or have not amplified the gene. Examples of dominant selection genes of this type include the genes for procaryotic enzymes such as xanthine-guanine phosphoribosyltransferase (Mulligan et al. "Proc. Nat. Acad. Sci." 78[4]:2072-2076 (1981) and aminoglycoside 3' - phosphotransferase (Colber-Garapin et al., "J. Mol. Biol.", 150:1-14 (1981).

Some dominant selection genes also are detectably amplified. Suitable examples include the mutant DHFR gene described by Haber et al., "Somatic Cell Genet." 4:499-508 (1982), cell surface markers such as HLA antigens and genes coding for enzymes such as specific esterases that produce fluorescent or colored products from fluorogenic or chromogenic substrates as is known in the art.

Detectably-amplified, dominant selection genes are preferred for use herein. It should be understood that a dominant selection gene in some cases can be converted to a detectably amplified gene by suitable mutations in the gene.

Selection genes at first were of limited commercial utility. While they enabled one to select transformants having the propensity to amplify uptaken DNA most selection genes produced products of no commercial value. On the other hand, genes for products which were commercially valuable generally did not confer readily selectable (or even detectable) phenotypes on their transformants. This would be the case, for example, with enzymes or hormones which do not provide transformed cells with unique nutrient metabolic or detoxification capabilities. Most proteins of commercial interest fall into this group, e.g. hormones, proteins participating in blood coagulation and fibrinolytic enzymes.

Subsequently it was found that eucaryotic cells having the propensity to be transformed with and amplify the selection gene would do the same in the case of the product gene. By following the selection gene one could identify a subpopulation of transformant cells which coexpress and coamplify the product gene along with the selection gene. It has been the practice to culture the transformants in the presence of the selection agent and to conclude that transformants having increased expression of the selection gene will also show increased expression of the product gene. This is not always the case, as will be more fully explored below. Axel et al. (U.S. patent 4,399,216) use the term cotransformation to describe the process of transforming a cell with more than one different gene, whether by vector systems containing covalently linked or unlinked genes, and in the latter case whether the genes are introduced into host cells sequentially or simultaneously. Cotransformation should "allow the introduction and stable integration of virtually any defined gene into cultured cells" (Wigler et al. "Cell", 16:777-785, (1975), and "by use of the cotransformation process it is possible to produce eucaryotic cells which synthesize desired proteinaceous and other materials" (U.S. patent 4,399,216, column 3, lines 37-42).

Transformation Vectors

Vectors used in AHF cotransformation will contain a selection gene and the AHF gene. In addition there usually will be present in the transformation or cotransformation vectors other elements sub as enhancers, promoters, introns, accessory DNA, polyadenylation sites and 3' noncoding regions as will be described below.

Suitable selection genes are described above. It is preferred that the selection agent be one that prevents cell growth in the absence of the selection gene. That way, revertant cells in large scale culture that lose the selection gene (and presumably the AHF gene as well) will not over-grow the fermentation. However, it would be desirable in the commercial production of AHF to avoid the use of cell toxins, thereby simplifying the product purification steps. Thus, a desirable selection gene would be one that enables transformants to use a nutrient critical for growth that they otherwise would not be able to use. The TK gene described above is an example.

Two classes of vectors have been employed in cotransformation. The first class are the unlinked vectors. Here the selection gene and the AHF gene are not covalently bound. This vector class is preferred because the step of ligating or otherwise bonding the two genes is not required. This simplifies the transformation process because the selection and product genes usually are obtained from separate sources and are not ligated in their wild-type environment. In addition, the molar ratio of the AHF and selection genes employed during cotransformation can be adjusted to increase cotransformation efficiency.

The second class of cotransformation vectors are linked vectors. These vectors are distinguished from unlinked vectors in that the selection and AHF genes are covalently bound, preferably by ligation.

The vectors herein may also include enhancers. Enhancers are functionally distinct from promoters, but appear to operate in concert with promoters. Their function on the cellular level is not well understood, but their unique characteristic is the ability to activate or potentiate transcription without being position or orientation dependent. Promoters need to be upstream of the gene, while enhancers may be present upstream or 5' from the promoter, within the gene as an intron, or downstream from the gene between the gene and a polyadenylation site or 3' from the polyadenylation site. Inverted promoters are not functional, but inverted enhancers are. Enhancers are cis-acting, i.e., they have an effect on promoters only if they are present on the same DNA strand. For a general discussion of enhancers see Khoury et al., "Cell" 33:313-314 (1983).

Preferred enhancers are obtained from animal viruses such as simian virus 40, polyoma virus, bovine papilloma virus, retrovirus or adenovirus. Viral enhancers may be obtained readily from publically available viruses. The enhancer regions for several viruses, e.g., Rous sarcoma virus and simian virus 40, are well known. See Luciw et al., "Cell" 33:705-716 (1983). It would be a matter of routine chemistry to excise these regions on the basis of published restriction maps for the virus in question and, if necessary, modify the sites to enable splicing the enhancer into the vector as desired. For example, see Kaufman et al, "J. Mol. Biol.", 159:601-621 (1982) and "Mol. Cell Biol." 2(11):1304-1319 (1982). Alternatively, the enhancer may be synthesized from sequence data; the sizes of viral enhancers (generally less than about 150 bp) are sufficiently small that this could be accomplished practically.

Another element which should be present in the vector assembly is a polyadenylation splicing (or addition) site. This is a DNA sequence located downstream from the translated regions of a gene, shortly downstream from which in turn transcription stops and adenine ribonucleotides are added to form a polyadenine nucleotide tail at the 3' end of the messenger RNA. Polyadenylation is important in stabilizing the messenger RNA against degradation in the cell, an event that reduces the level of messenger RNA and hence the level of product protein.

Eucaryotic polyadenylation sites are well known. A consensus sequence exists among eucaryotic genes: The hexanucleotide 5'-AAUAAA-3' is found 11-30 nucleotides from the point at which polyadenylation starts. DNA sequences containing polyadenylation sites may be obtained from viruses in accord with published reports. Exemplary polyadenylation sequences can be obtained from mouse beta-globulin, and simian virus 40 late or early region genes, but viral polyadenylation sites are preferred. Since these sequences are known, they may be synthesized in vitro and ligated to the vectors in conventional fashion.

A polyadenylation region must be located downstream from either the AHF and/or selection gene, but may be ligated to either gene. It may be ligated to the selection gene only, and not the product gene, and this will be the case whether the vectors are linked or unlinked. The sequence which separates the polyadenylation site from the translational stop codon is preferably an untranslated DNA oligonucleotide such as an unpromoted eucaryotic gene. Since such oligonucleotides and genes are not endowed with a promoter they will not be expressed. The oligonucleotide should extend for a considerable distance, on the order of up to about 1,000 bases, from the stop codon to the polyadenylation site. This 3' untranslated oligonucleotide generally results in an increase in product yields. The vector may terminate from about 10 to about 30 bp downstream from the consensus sequence, but it is preferable to retain the 3' sequences found downstream from the polyadenylation site in its wild-type environment. These sequences typically extend about from 200 to 600 base pairs downstream from the polyadenylation site.

The vectors described herein may be synthesized by techniques well known to those skilled in this art. The components of the vectors such selection genes, enhancers, promoters, and the like may be obtained from natural sources or synthesized as described above. Basically, if the components are found in DNA available in large quantity, e.g. components such as viral functions, or if they may be synthesized, e.g. polyadenylation sites, then with appropriate use of restriction enzymes large quantities of vector may be obtained by simply culturing the source organism, digesting its DNA with an appropriate endonuclease, separating the DNA fragments, identifying the DNA containing the element of interest and recovering same. Ordinarily, a transformation vector will be assembled in small quantity and then ligated to a suitable autonomously replicating synthesis vector such as a procaryotic plasmid or phage. The pBR322 plasmid may be used in most cases. See Kaufman et al., op. cit.

The synthesis vectors are used to clone the ligated transformation vectors in conventional fashion, e.g. by transfection of a permissive procaryotic organism, replication of the synthesis vector to high copy number and recovery of the synthesis vector by cell lysis and separation of the synthesis vector from cell debris.

The resulting harvest of synthesis vector may be directly transfected into eucaryotic cells, or the transformation vector may be rescued from the synthesis vector by appropriate endonuclease digestion, separation by molecular weight and recovery of the transformation vector. Transformation vector rescue is not necessary so long as the remainder of the synthesis vector does not adversely affect eucaryotic gene amplification, transcription or translation. For example, the preferred synthesis vector herein is a mutant of the E. coli plasmid pBR322 in which sequences have been deleted that are deleterious to eucaryotic cells. See Kaufman et al., op. cit. Use of this mutant obviates any need to delete the plasmid residue prior to cotransformation.

Cotransformation, Selection and Detection of Amplification

The cells to be transformed may be any eucaryotic cell, including yeast protoplasts, but ordinarily a nonfungal cell. Primary explants (including relatively undifferentiated cells such as stem cells), and immortal and/or transformed cell lines are suitable. Candidate cells need not be genotypically deficient in the selection gene so long as the selection gene is dominant acting.

The cells preferably will be stable mammalian cell lines as is discussed above. Cell lines that are known to stably integrate selection genes into their chromosomal DNA are best, for example Chinese hamster ovary (CHO) cell lines. Also useable are HeLa, COS monkey cells, melanoma cell lines such as the Bowes cell line, mouse L cells, mouse fibroblasts and mouse NIH 3T3 cells.

Cotransformation with unlinked vectors may be accomplished serially or simultaneously, (see U.S. patent 4,399,216). Methods for facilitating cellular uptake of DNA are described above. Microinjection of the vector into the cell nucleus will yield the highest transformation efficiencies, but exposing parental cells to DNA in the form of a calcium phosphate precipitate is most convenient. Considerably better cotransformation efficiencies result from cotransformation with a molar excess of product to selection gene, on the order of 100:1.

The population of cells that has been exposed to transforming conditions is then processed to identify the transformants. Only a small subpopulation of any culture which has been treated for cotransformation will exhibit the phenotype of the selection gene. The cells in the culture are screened for the phenotype. This can be accomplished by assaying the cells individually with a cell sorting device where the phenotype is one that will produce a signal, e.g. fluorescence upon cleavage of a fluorogenic substrate by an enzyme produced by the selection gene. Preferably, however, the phenotype enables only transformants to grow or survive in specialized growth media as is further discussed above.

Selection transformants then will be screened for ligation of the product gene into their chromosomes or for expression of the product itself. The former can be accomplished using Southern blot analysis, the latter by standard immunological or enzymatic assays.

Once the tranformants have been identified, steps are taken to amplify expression of the product gene by further cloning in the presence of a selection agent such as MTX. See U.S. Patent 4,399,216.

Cotransformants which may be produced in accordance with the processes described herein are suitable for in vivo transfections of higher organisms in accordance with known techniques. Primary explants or stable cell lines from a potential host animal are cotransformed and inoculated into the host or a substantially otherwise syngeneic host which is genotypically deficient in the product protein.

The invention will be further understood with reference to the following illustrative embodiments, which are purely exemplary, and should not be taken as limitive of the true scope of the present invention, as described in the claims.

Unless otherwise noted, restriction endonucleases are utilized under the conditions and in the manner recommended by their commercial suppliers. Ligation reactions are carried on as described by Maniatis et al., Molecular Cloning, A Laboratory Manual, (Cold Spring Harbor Laboratory 1982) at 245-6, the disclosure of which is incorporated herein by reference, using the buffer described at page 246 thereof and using a DNA concentration of 1-100 ug/ml, at a temperature of 23°C for blunt ended DNA and 16°C for "sticky ended" DNA. "Phosphatasing" as described herein, refers to dephosphorylation of DNA, and is carried out in the manner described by Maniatis et al., supra, e.g. at page 133 et seq. "Kinasing" refers to phosphorylation of DNA. Electrophoresis is done in 0.5-1.5% Agarose gels containing 90 mM Tris-borate, 10 mM EDTA. "Nick-translating" refers to the method for labeling DNA with 32p as described by Rigby et al., J. Mol. Biol., 113:237 (1977). All radiolabeled DNA is labeled with $^{32}$P, whatever labeling technique was used.

By "rapid prep" is meant a rapid, small scale production of bacteriophage or plasmid DNA, e.g., as described by Maniatis et al., supra, at p. 365-373.

In accordance with another aspect of this invention, there is provided a pharmaceutical preparation of AHF. A pharmaceutical preparation of human AHF produced in accordance with this invention may be prepared for parenteral administration in accordance with procedures well known in the art.

The pharmaceutical preparation for human use comprises sterilized AHF recovered from transformed cells. In addition to the AHF polypeptide or sufficient portion thereof which produces AHF activity, there may be included one or more acceptable carriers therefor and optionally other therapeutic ingredients. The carrier(s) must be "acceptable" in the sense of being compatible with other ingredients of the preparation and not deleterious to the recipient thereof. The preparation may conveniently be presented in unit dosage form and may be prepared by any of the methods well known in the art of pharmacy.

The pharmaceutical preparation of this invention, suitable for parenteral administration, may conveniently comprise a sterile lyophilized preparation of the AHF polypeptide which may be reconstituted by addition of sterilized solution to produce solutions preferably isotonic with the blood of the recipient. The preparation may be presented in unit or multi-dose containers, for example sealed ampoules or vials. It should be understood that in addition to the sterile

AHF or solution thereof, the pharmaceutical preparation of this invention may include one or more additional ingredients such as diluents, buffers, binders, surface active agents, thickeners, lubricants, preservatives (including anti-oxidants) and the like. Gelatine, lactose, starch, magnesium sterate, micronized silica gel, cocoa butter, talc, vegetabilic and animalic fats and oils, vegetabilic rubber and polyalkylene glycol and other known carriers for pharmaceuticals are all suitable for manufacturing the pharmaceutical preparations of the present invention. Preparations for parenteral use include an ampoule of a sterile solution or suspension with water or other pharmaceutically acceptable liquid as the carrier therefor, or an ampoule of sterile solid AHF for dilution with a pharmaceutically acceptable liquid.

The pharmaceutical preparation of the present invention is useful in the treatment of Hemophilia A. These preparations also provide an important tool in the in vivo as well as the in vitro study of the processes involved in clotting and in the study of the immunological and biological characteristics of the AHF molecule.

## Example 1. Protein Sequence Analysis

Porcine factor VIII:C was purified by Dr. David Fass according to published procedures, Knutsen and Fass (1982); Fass et al. (1982), supra. Amino acid sequence analysis is performed on a bovine thrombin digest product of the 76,000 dalton protein as described below.

Porcine AHF polypeptides bound to the anti-VIII:C monoclonal antibody column, can be sucessively eluted in two steps (Fass et.al. 1982). The two larger molecular weight species, 166,000 and 130,000 daltons, can be eluted with EDTA. The remaining polypeptide, having a molecular weight of about 76,000 daltons, can then be eluted with 50% ethylene glycol.

The 76,000 dalton protein is digested with thrombin after extensive dialysis in 50 mM Tris-HCl (pH 7.5), 0.15 M NaCl. Bovine thrombin digests are performed at room temperature for 60 minutes using 1 unit/ml of bovine thrombin, followed by the addition of another 1 unit/ml thrombin and incubation for an additional 60

minutes. The thrombin digestions are terminated by heating for 10 minutes at 9O°C in 0.O1% SDS. The major thrombin digest product of the 76,000 dalton protein is a polypeptide, 69,000 daltons (69 Kd).

Less than 1 ug of the 69 Kd polypeptide species described above is iodinated to serve as a radioactive marker after SDS gel electrophoresis, in accordance with the procedure of U. K. Laemmli, Nature, 227:680 (1970). The polypeptide, dissolved in TAS buffer (50 mM Tris-acetate (pH 7.8), 0.1% SDS) is added to 100 ul of the same buffer containing 5 mCi of carrier-free iodine-125. 50 ul of 2.5 mg/ml chloramine T (Baker) in TAS buffer are added and the solution agitated for 1 minute. The reactions are stopped by adding 50 ul 2.5 mg/ml sodium metabisulfate in TAS buffer followed by 1 minute agitation. Labeled protein is separated from unincorporated $^{125}$I by chromatography using a small volume Sephadex G-25 M column (PD-10, Pharmacia). The column is pre-equilibrated with several column volumes of TAS buffer containing 2.5 mg/ml sodium iodide. The void volume is collected and protein integrity analyzed by SDS gel electrophoresis in accordance with the procedures of Laemmli, (197O), supra.

The radioactively labeled 69 Kd protein is added to its unlabeled counterpart for subsequent monitoring. The protein is then individually electrophoretically concentrated. The protein solutions are adjusted to 0.1% SDS, 10 mM dithiothreitol and Coomassie brilliant blue (Serva) are added to make the solution a very pale blue.

The solutions are then dialyzed briefly (1-2 hours) in TAS buffer using Spectrapore dialysis tubing (made by Spectrum Medical Industries, Inc., with molecular weight cut off at about 14,000). The dialyzed protein samples are then placed in an electrophoretic concentrator, of the design suggested by Hunkapiller, et al., Meth. Enzymol., Enzyme Structures, Part I, 91:227 (1983), and electrophoretically concentrated in TAS buffer for 24 hours at 50 volts.

The 69 Kd AHF polypeptide, concentrated by the above procedure, is electrophoresed through an SDS-polyacrylamide gel in accordance with Laemmli, supra. The purified protein, identified by autoradiography of the radioactively labeled tracer polypeptide, is excised from the gel and electroeluted and concentrated as descibed by Hunkapiller et al., supra. The concentrated sample is suitable for direct amino acid sequence analysis using the gas phase sequenator as described in Hewick et al., J. Biol. Chem. 256:7990 (1981).

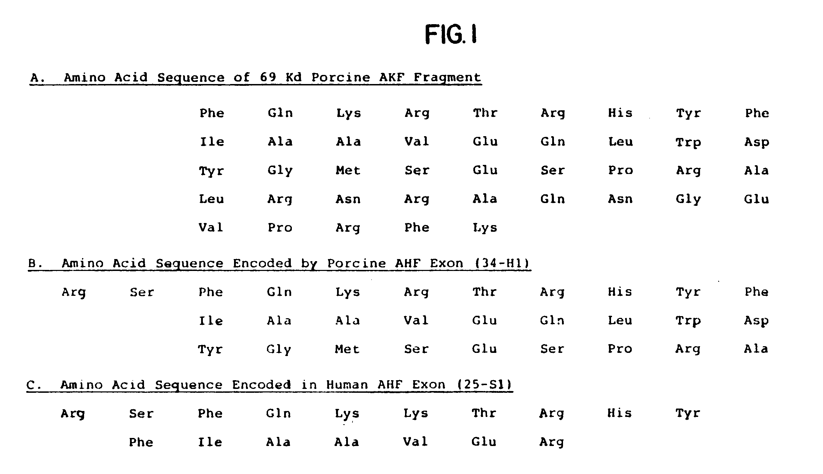

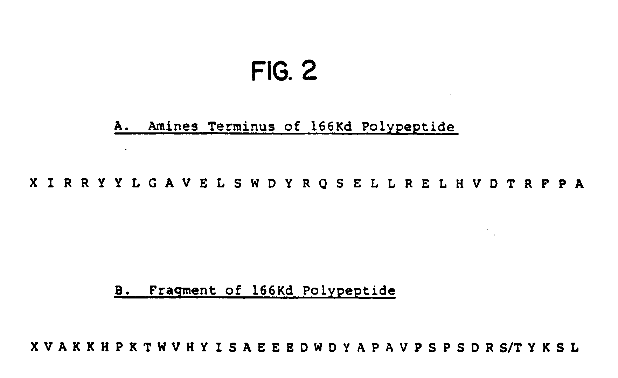

The amino terminal sequence extending from the 2-42 residue of the 69,000 dalton thrombin cleavage product is as depicted in Figure 1. The first residue "X" was not identifiable. The amino acid sequences of (A) the amino terminus and (B) a 40 kd bovine thrombin digestion product from the 166 kd AHF fragment noted by Fass et al., supra, are shown in Figure 2. The amino acid sequence of the amino terminus of the 76 Kd polypeptide is: X-Ile Ser Leu Pro Thr Phe Gln Pro Glu Glu Asp Lys Met Asp Tyr Asp Asp Ile Phe.

Example 2

Chemical Synthesis of Oligonucleotide Probes for a Porcine AHF Gene

(a) Pentapeptide Probe Pool

The partial amino acid sequence of a fragment of porcine AHF having been determined allows porcine AHF oligonucleotide probes to be designed and synthesized. From the genetic code (Table 1) it is possible to predict the gene sequence that codes for this sequence of amino acids. Because the genetic code is degenerate, there are more than one possible DNA coding sequences for each amino acid sequence. Accordingly, a plurality or pool of complementary oligonucleotide probe sequences are provided for a region of the AHF molecule which required only a reasonable number of oligonucleotides to ensure the correct DNA sequence. Such regions are selected by searching for tracks of five to eight contiguous amino acids which have the lowest degeneracy. After the region is selected, a pool of oligonucleotides is synthesized, which would include all possible DNA sequences which could code for the five to eight amino acids in the selected region.

In the 69,000 dalton thrombin-cleavage fragment of porcine AHF there is a pentapeptide sequence running from the 18th through the 22nd amino acid from the amino terminus, which could be coded for by up to 16 different DNA sequences, each having five codons, for a length of 15 nucleotides.

11

| | | 18 | | 20 | | 22 |
|---|---|---|---|---|---|---|
| | | Trp – | Asp – | Tyr – | Gly – | Met |
| Possible mRNA Sequences | | UGG | GAU | UAU | GGU | AUG |
| | | | or | or | or | |
| | | | GAC | UAC | GGC | |
| | | | | | or | |
| | | | | | GGA | |
| | | | | | or | |
| | | | | | GGG | |

The probes are made by synthesizing a limited number of mixtures of oligonucleotides with two to eight or more oligonucleotides per mixture. These mixtures are referred to as pools. Enough pools are made to encompass all possible coding sequences.

These oligonucleotides can be synthesized manually, e.g., by the phospho-tri-ester method, as disclosed, for example in R. L. Letsinger, et al., J. Am. Chem. Soc. 98:3655 (1967). Other methods are well known in the art. See also Matteucci and Caruthers, J. Am. Chem. Soc. 103:3185 (1981).

Preferably, however, the synthetic oligonucleotide probes for the desired polypeptide sequences are prepared by identical chemistry with the assistance of the completely automatic Applied Biosystems DNA synthesizer, Model 380A, as indicated above.

The oligonucleotides thus prepared can then be purified on a reverse HPLC column, as described by H. Fritz, et al., Biochemistry, 17:1257 (1978). After detritylation with 80% HOAc, the resulting oligonucleotide is normally pure and can be used directly as a probe. If there are any contaminants, the synthetic DNA can be further purified on the same HPLC column, preferably using a slightly different gradient system.

Oligonucleotides are labelled, e.g. by using [$\gamma$-$^{32}$P]ATP and T4 polynucleotide kinase, and their sequence checked either by two-dimensional homochromatography as described by Sanger et al., PNAS U.S.A. 70:1209 (1973) or by the Maxam-Gilbert method, Meth. Enzymology, 65:499 (1977).

(b) Forty Five Nucleotide Probes

A unique aspect of the present invention has been the use of oligonucleotide probes to screen a genomic DNA library for the AHF gene or fragments thereof. While oligonucleotides have been used for screening of cDNA libraries, see, e.g. M. Jaye, et al, Nucleic Acids Research 11:2325 (1982) genomic libraries have previously been screened successfully only with cDNA probes, i.e. probes which were generated only after the tissue source of the mRNA for a described protein had been identified and utilized to generate a cDNA clone which precisely matched the DNA sequence of the gene sought by the genomic search.

In the present case, it has been shown possible to use oligonucleotides to identify gene segments in a genomic library which code for the amino acid sequence of the proteins of interest, and identification of such gene segments provides an exact probe for use in mRNA, cDNA or further genomic screening techniques.

Preferably, as here, oligonucleotides corresponding to at least two segments of the amino acid sequence of the protein of interest are utilized. Preferably at least one of the oligonucleotide probes is used in the form of one or more pools of oligonucleotides which in the aggregate include every possible DNA sequence which could code for the amino acid sequences selected. Preferably one relatively short probe, e.g. 11 to 25 nucleotides, preferably 15 to 20 nucleotides is utilized in conjunction with a relatively long probe, e.g. 30 to 200 nucleotides, preferably 40 to 50 nucleotides. The second probe can be used for confirmation, and is not always necessary for identification of the DNA segment. Preferably at least one of the probes, and more preferably the longer of the probes is designed in accordance with the Rules 1 to 4 described below.

Rule 1. Codon Preference In the absence of other considerations, the nucleotide sequence was chosen which matched prevailing or similar sequences in similar mammalian genes. See Mechanisms of Ageing Dev., 18: (1982).

Rule 2. Advantageous GT Pairing The nucleotide G, in addition to bonding to its complement C, can also form weak bonds with the nucleotide T. See K. L. Agarwal et al., J. Biol. Chem. 256:1023 (1981). Thus, faced with a choice of G or A for the third portion of an ambiguous codon, it is preferable to choose G, since if the resulting hybridization would occur even if the actual nucleotide in the position is a T, rather than a C, the hybridization would still be stable. If an A were chosen incorrectly, the corresponding A:C incompatibility could be enough to destroy the ability of the probe to hybridize with the genomic DNA.

Rule 3. Avoidance of 5'OG Sequences

When selecting from among the possible ambiguities, select those nucleotides which will not contain the 5'CpG sequence, either intra codon or inter codon.

Rule 4. Mismatch Position In choosing the codon sequences to use, consideration was given to the postulation that mismatches near the ends of the molecule do not adversely affect the stability of the hybridization as do mismatches near the center of the molecule. Thus, for example, where substantial doubt occurred concerning the sequences of a particular codon, and that codon was close to the center of the probe, the tendency was to test a pool of possible nucleotide sequences for that codon, whereas codon positions near the ends of the probe were more likely to be subject to determinations on the basis of codon preference.

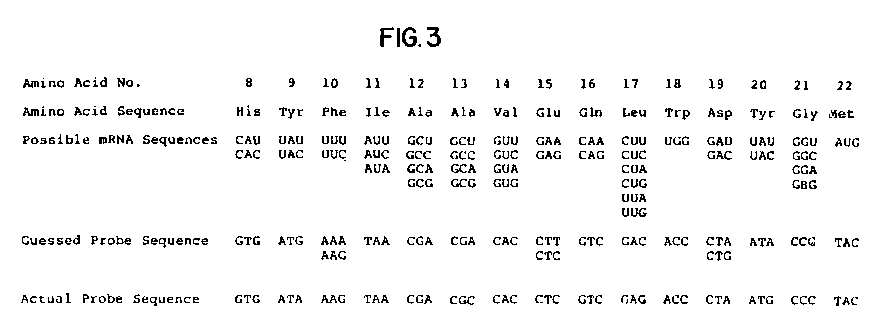

The chosen sequence for the 45-mer probes, as well as the amino acid sequences, the possible DNA sequences, and the "Actual Probe Sequence", i.e. the complement of actual coding strand determined for the AHF exon are shown in Figure 3.

Thus, out of nucleotide positions involving choices, three were covered by using pools containing both possible nucleotide alternatives, five were predicted correctly, one was predicted in a way to maintain neutrality though incorrect, and four others were in error.

Despite the approximately 11% mismatch (5/45) the pool of 45-mer oligonucleotides are adequate to strongly identify a porcine AHF gene fragment, as described below.

Example 3. Screening of Porcine Genomic Library

A porcine genomic library is constructed using the bacteriophage vector Lambda J1. Lambda J1 is derived from L47.1 (Loenen et al., Gene 20:249 (1980)) by replacement of the 1.37 kb and 2.83 kb Eco RI-Bam HI fragments with a 95 bp Eco R1-Hind III-Xba I-Bgl II-Bam HI polylinker. The 6.6 kb Bam HI fragment is then present as a direct repeat in reverse orientation relative to L47.1. The cloning capacity for Bam HI fragments is 8.6 - 23.8 kb. Bam HI cleaved porcine DNA (prepared as described by Piccini et al., Cell, 30:205 (1982)) is phenol extracted, ethanol precipitated and concentrated by centrifugation in a Microfuge. 0.67 ug of Bam HI porcine DNA is ligated to 2 ug of Lambda J1 Bam HI "arms", prepared as described in Maniatis, et al., supra, pp 275-279, in a volume of 10 ul ligation buffer with 10 units T4 DNA ligase (Maniatis et al., supra, p 474). The ligated DNA is packaged and plated as described in Maniatis et al., supra, p 291.

Approximately 4 x 105 pfu are plated on E. coli strain C600 on 15 cm plastic petri plates containing NZCYM agarose, at 8,000 pfu/plate. These recombinant phage are screened by the method of Woo (1979) using the 45-mer probe described above, radioactively labeled with $^{32}$p as described above, as a probe. Filters are then hybridized in 5xSSC, 5x Denhardt's, 0.1% SDS and 5 x 10$^6$ cpm/ml probe at 45° C for 16 hours, washed in 5 x SSC, 0.1% SDS at 50° C and subjected to autoradiography using intensifying screens (DuPont Lightning-Plus). Autoradiography reveals numerous phage which hybridized, to varying degrees, with the 45-mer. The filters are then denatured in 0.5M NaOH, neutralized in 1.0M Tris pH7.5, 1.5M NaCl and hybridized to the 15-mer as described for the 45-mer except the hybridization and washing temperature is 37° C. One phage which hybridized to both probes is picked from the original plate and 100 pfu plated and the plaques screened as described above using the 15-mer as probe.

A positive phage, named PB34, is picked as a plug and used to make a plate lysate as described in Maniatis et al, supra, pp 65-66. A small-scale isolation of PB34 DNA is achieved using the procedure described in Maniatis et al., supra, pp 371-372. 10ul of this DNA was cut with the restriction enzyme Hae III and then phosphatased using calf alkaline phosphatase (Boehringer-Mannheim). After phenol extraction, 20 ng of Sma I cut M13mp8 DNA is added, the solution is made 0.2M NaCl and nucleic acid precipitated by the addition of 2 volumes ethanol. Precipitated DNA is pelleted by centrifugation and redissolved in 2 ul of a ligation mixture and the DNA is ligated for 30 minutes at 23° C, diluted to 50 ul with ligase buffer and ligated an additional 3 hours. 5 ul of this reaction is used to transform E. coli strain JM101/TG1.

Cells are made competent for transformation by growing to an 0.D.600 of 0.5 at 37° C in 50 ml SOBM media (SOBM is 2% tryptone, 0.5% yeast extract, 0.1M NaCl, 0.11g KOH per liter, 20mM MgSO$_4$). Cells are pelleted by centrifugation at 2500 rpm for 10 minutes at 4° C. The cells are resuspended in 3.5 ml 100mM RbCl, 45mM MnCl$_2$, 50mM CaCl$_2$, 10mM potassium MES pH 6.4 (MES = methylethane sulfonic acid). 200ul of competent cells are transformed with the DNA contained in 5 $\mu$l of the ligation reaction at 0° C for 30 minutes. The cells are then heat-shocked at 42° C for 90 seconds after which 4 ml of 0.8% agarose/SOBM containing 100 ul stationary JM101/TG1 cells are added and plated on 10 cm SOBM agar petri plates.

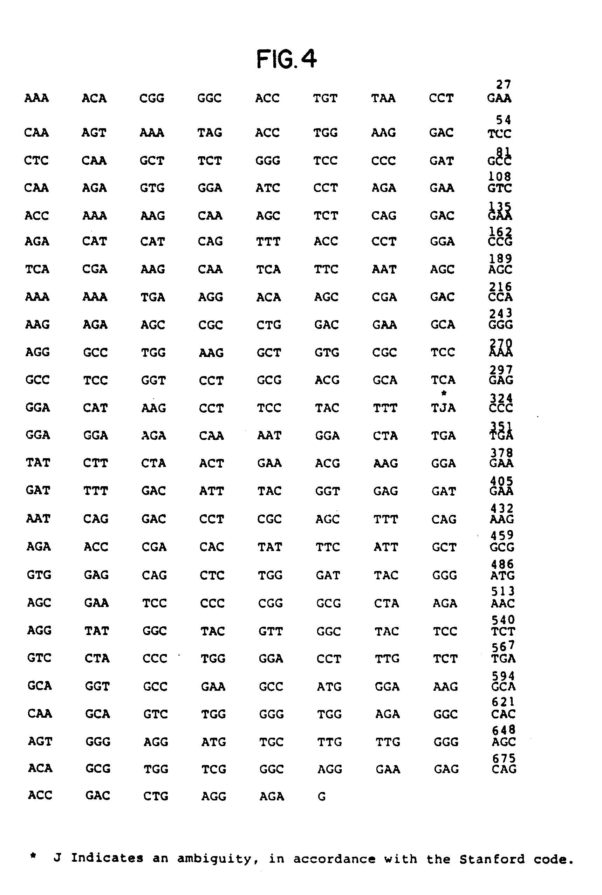

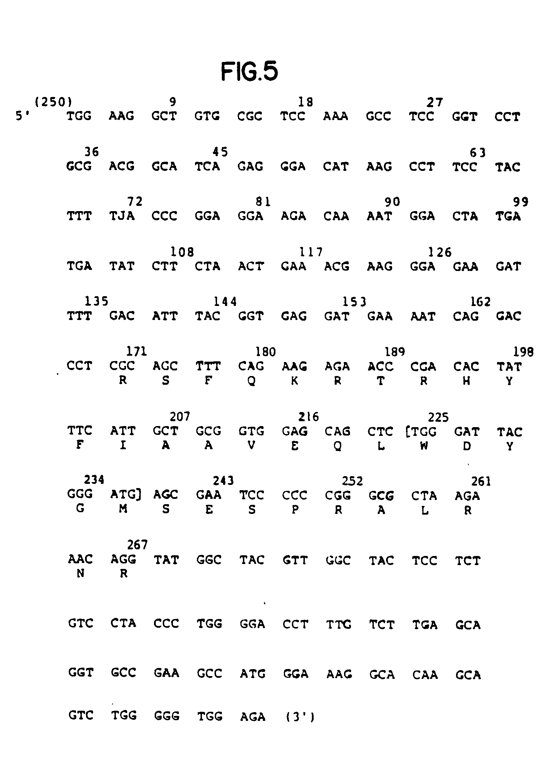

A subclone, containing a Hae III fragment from PB34, hybridizing to the 15-mer is identified by screening using the procedure of Benton and Davis, supra. This clone is isolated and prepared for use as a template. The DNA sequence of the Hae III fragment present in this clone, designated as 34-H1, is shown in Figure 5. M13 template DNA is prepared by growing 1.5 ml of infected cells 5 hours at 37° C. Cells are pelleted by centrifugation for 10 minutes in a Beckman microfuge. 1.0 ml of supernatant (containing virus) is removed and 200 ul of 20% polyethylene glycol, 2.5 M NaCl is added. This sample is then incubated at room temperature for 15 minutes followed by centrifugation for 5 minutes in a Beckman microfuge. The pellet is dissolved in 100 ul TE, 7.5 ul of 4M NaCHCOO pH 4.5 is added and the sample extracted twice with a 1:1 mixture of phenol-chloroform and once with chloroform. The single-stranded phage DNA is then precipitated by the addition of 2 volumes of ethanol. Precipitated DNA is pelleted by centrifugation in a Beckman microfuge and dissolved in 30 ul 1mM Tris pH 8.0, 0.1 mM EDTA. The DNA sequencing is performed by the dideoxy chain termination technique, see, e.g. Sanger et al., PNAS U.S.A., 74:5463 (1977), utilizing the 15-mer as a primer. The sequence observed, which is included in the sequences represented in Figs. 4 and 5, confirmed the subclone as containing a porcine AHF exon as it includes the identical 14 amino acids encompassed by the region phenylalanine$_2$ to glutamine$_{15}$ in the amino terminal sequence of the 69Kd fragment represented in Figure 1. Further confirmation came from sequencing from the 5' end of the Hae III insert in the 34-H1 vector, by priming with the "Universal primer" (Bethesda Research Labs) at a point adjacent the polylinker in that vector. Also, the insert from this clone, named 34-H1, was recloned into M13mp9 by restricting the DNA with Eco RI and Hind III, phosphatasing with calf alkaline phosphatase, and ligating to Eco RI, Hind III cleaved M13mp9 DNA. This clone, which contains an inversion of the Hae III segment relative to the universal primer, was also sequenced as described above. The resulting sequence data for all of insert 34-H1 is shown in Figure 5. This sequence confirms that this subclone contains an exon of the porcine AHF gene that could encode, from nucleotides 169 to 267, at least the thirty amino acids from the phenylalanine$_2$ through arginine$_{31}$ of the 69 Kd fragment (Fig. 1).

It appears likely that arginine$_{31}$ borders because termination codons can be found downstream from nucleotide 267 (Fig. 5) in all three reading frames, and a sequence similar to the consensus 5' splice site sequence is also found in that region between nucleotides 266-267. Further, the amino acid sequence which would be encoded by the downstream DNA differs completely from that observed in the 69 Kd fragment of porcine AHF.

PB34 DNA was cut with Bam HI and electrophoresed through an agarose gel and the bands visualized by ultraviolet light after staining the gel in 5 μg/ml ethidium bromide. Three inserts of approximately 6.6 kb, 6.0 kb, and 1.8 kb were observed. The DNA in the gel was transferred to nitrocellulose as described in Maniatis et al., supra, pp 383-386. Hybridization of the filter to the 15-mer and autoradiography were performed as described above. Autoradiography revealed that the 6.0 kb band contained an AHF gene fragment which hybridized to the 15-mer probe.

Thus, for the first time, a section of the gene coding for porcine AHF had been isolated and identified. The bacteriophage lambda recombinant clone PB 34 is on deposit at the American Type Culture Collection under Accession Number ATCC 40087.

Example 4. Location of Human AHF Gene

The human genomic library described by Maniatis et al., Cell, 15:687 (1978) is screened for the human AHF gene by infecting E. coli strain LE392 (publicly available) with 6x10$^5$ pfu and plating on 15cm NZCYM agar plates at a density of 20,000 pfu/plate. These phage are screened using the procedure of Benton and Davis, supra with the 6.0 kb porcine AHF fragment described in Example 3, labeled with $^{32}$P by nick translation, as the probe. A phage exhibiting a strong hybridization signal is picked and plated at about 100 pfu/10cm plate and screened in duplicate as described above using the radioactively labeled 45-mer as one probe and the 6.0 kb Bam HI fragment of PB34 as the other. A phage, named HH25, which hybridizes to both probes is identified, a plate stock made and rapid prep DNA prepared as described above. The phage DNA is cut with Sau3A I, phosphatased with calf alkaline phosphatase, phenol extracted, and co-precipitated with 20 ng of Bam HI cut M13 mp8 DNA. Precipitated DNA is pelleted by centrifugation and redissolved in 2 ul ligase buffer containing T4 DNA ligase. Ligation is performed for 2 minutes at 16° C, diluted to 50 ul in ligase buffer containing T4 DNA ligase and incubated an additional 3 hours at 16° C. 5 ul of this reaction mixture is used to transform E. coli strain JM101/TG1 as described in Example 3 above.

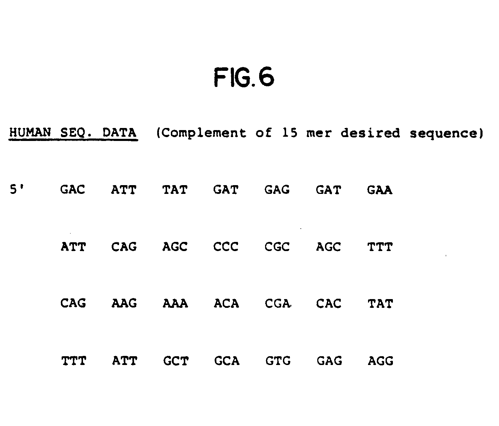

The plaques are screened using the Benton and Davis procedure and probing with radioactively labeled 15-mer. A phage plaque, named 25-S1, exhibiting hybridization is isolated and single-stranded phage DNA prepared for use as a DNA sequencing template as described above. Sequencing is performed using the dideoxy chain termination technique described by Sanger et al., supra, utilizing the 15-mer as primer, and gives the information strand DNA sequence shown in Figure 6. 84 nucleotides sequenced in this manner demonstrated 85% homology with the homologous region of porcine AHF. There is also only one amino acid difference between the procine AHF 69 Kd region 2-16 as shown in Figure 1 and the corresponding region which was deduced from the human nucleotide sequence of Figure 6. This high degree of homology shows that the DNA of the recombinant phage HH-25 emanates from the AHF gene.

Thus, for the first time, an exon for the human AHF gene has been isolated and identified. A bacteriophage lambda recombinant HH 25 is on deposit at the American Type Culture Collection under the Accession Number ATCC 40086.

Example 5 Identifying Cells Actively Transcribing AHF

The porcine and human AHF exons described above are useful for a variety of functions, one of which is as a screening agent which permits identification of the tissue which is the site of synthesis of AHF in vivo. A number of screening methods are available, based on the use of the exon as an exact complement to the mRNA which is produced during the course of natural expression of AHF. In the screening procedures, tissue from various parts of the body is treated to liberate the mRNA contained therein, which is then hybridized to a DNA segment containing the exon for AHF, and if a molecule of mRNA does hybridize to that exon, the tissue which is the source of that mRNA is the source of AHF.

1. Screening Procedure

Porcine or human tissue from various organs, including kidneys, liver, pancreas, spleen, bone marrow, lymph nodes, etc., is prepared by guanidine hydrochloride extraction as described by Cox Methods Enzymol., 12B:120 (1968) with some modifications. Briefly, tissue is explanted into 8 M guanidine hydrochloride (or 4M guanidine isothiocyanate as proposed by Chirgwin, et al., Biochemistry 18: 5294 (1979), see also Maniatis, et al., supra, at 189 et seq.), 50 mM Tris (pH 7.5), 10 mM EDTA and homogenized in an Omnimixer (Sorvall) at top speed for 1 minute. The homogenate is clarified at 5000 rpm for 5 minutes in a Sorval HB-4 rotor and the RNA is precipitated by the addition of 0.5 volumes of ethanol. The RNA is dissolved and precipitated 3 more times from 6 M guanidine hydrochloride before being dissolved in $H_2O$.

Messenger RNA from this pool is enriched by chromatography on oligo (dT) cellulose (Collaborative Research).

This mRNA is then subjected to electrophoresis through an agarose gel containing formaldehyde, as described by Maniatis et al., supra, at 202-3. The mRNA in the gel is then transferred to a nitrocellulose filter (Maniatis et al., supra, at 203-4).

The thus obtained mRNA is hybridized with the radiolabeled porcine or human exon DNA obtained as described above, and the existence of hybrids detected by autoradiography. A radioactive signal indicates that the tissue source of the mRNA is a source of synthesis of AHF in the body.

Alternatively, mRNA can be screened using the $S_1$ protection screening method.

$S_1$ nuclease is an enzyme which hydrolyzes single stranded DNA, but does not hydrolyze base paired nucleotides, such as hybridized mRNA/DNA. Thus the existence of a radioactive band after acrylamide gel electrophoresis and autoradiography shows that the single stranded DNA corresponding to the AHF exon has been protected by the complementary mRNA, i.e. the mRNA for AHF. Thus the tissue which was the source of that mRNA is a site of synthesis of AHF in vivo. That tissue can be used as a source for AHF mRNA. This method of screening can be somewhat more sensitive than screening method described above.

A probe consisting of single stranded radioactively labeled DNA complementary to the mRNA is synthesized using the universal primer of M13 to prime DNA synthesis of the porcine genomic subclone 34-H1. The reaction is performed in a 100 ul solution of 50 mM Tris pH 7.4, 5 mM $MgCl_2$, 1 mM 2-mercaptoethanol, 50 mM NaCl, 40 uM dGTP, dTTP, dCTP, 60 uCi of $^{32}P$-dATP (400Ci/mmole), 10 ng universal primer, 200-400 ng of 34-H1 template DNA, and the Klenow fragment of DNA polymerase I (E coli). The reaction is incubated at 23° C for 60 minutes, 10 minutes at 70° C, 50 units of PstI added and incubated an additional 60 minutes.

The reaction is terminated by phenol/chloroform extraction, NaCl added to 0.2M and then precipitated with two volumes 100% ethanol. The precipitated DNA is pelleted by centrifugation, redissolved in 20% sucrose, 50mM NaOH, 0.1% cresol green and then electrophoresed through 2% agarose in 50 mM NaOH, 10 mM EDTA. The resulting single stranded fragment is localized by autoradiography, the band excised and DNA isolated by electroelution in dialysis tubing.

Sample mRNA is prepared from liver, spleen, etc. tissue by the guanidine hydrochloride method described above.

The probe is then hybridized to sample mRNA (obtained from the oligo (dT) chromatography enriching step) in 50% formamide, 0.4M NaCl, 40 mM PIPES [piperazine-N,N' bis(2-ethanesulfonic acid)] pH 6.5, 1 mM EDTA, 5-50 ug mRNA, 2 ug labeled DNA in a volume of 15 ul. The hybridization is terminated by the addition of 200 ul cold S1 nuclease buffer (0.25 M NaCl, 0.3 M NaCH$_3$COO[pH 4.5], 1 mM ZnSO$_4$, 5% glycerol and 1000 units S$_1$ nuclease. The reaction is incubated at 45° C for 30 minutes. Samples are phenol extracted, ethanol precipitated with 10 ug yeast tRNA carrier and subjected to analysis by electrophoresis through 5% polyacrylamide sequencing gels as described in Maxam-Gilbert, PNAS USA, 74:560 (1977).

## Example 6 Use of AHF Exon DNA to Obtain AHF mRNA From Tissue

Once a tissue which is a source of mRNA is identified, AHF mRNA from that tissue is extracted and used to construct a cDNA library. The cDNA library is then utilized for identifying and constructing a full length cDNA clone which encodes the amino acid sequence for AHF, without the introns contained in the genomic clone,as described below. Thereafter, the cDNA which encodes the AHF protein is inserted into an appropriate expression vector, in an appropriate host, for expression of AHF.

Since human AHF is in great demand for treatment of hemophilia and other uses, the preparation of human AHF cDNA is described.

## 1. Obtaining mRNA for Human AHF

mRNA from the human tissue responsible for AHF synthesis is prepared by the guanidine hydrochloride extraction method as described by Cox and modified by Chirgwin et al. as disclosed above.

Further fractionation of mRNA obtained from the oligo (dT) cellulose chromatography column is obtained by sedimenting on 5-20% sucrose gradients containing 10 mM Tris-HCl (pH 7.4), 1mM EDTA and 0.2% SDS by centrifugation for 24 hours at 22,000 rpm in a Beckman SW28 rotor. Fractions (1.0 ml) are collected, sodium acetate was added to 0.2M, and the fractions are ethanol precipitated twice before dissolving in water. The size distribution of the fractionated RNA is determined by electrophoresis through 1.4% agarose gels containing 2.2 M formaldehyde.

mRNA sedimenting with an S (Svedberg) value greater than 28 is pooled for the synthesis of double stranded cDNA. 10 ug of this RNA is denatured at room temperature in 10 ul of 10 mM methylmercuryhydroxide. 140 mM 2-mercaptoethanol is added to inactivate the methylmercuryhydroxide. The RNA is then diluted to 50 ul containing 140 mM KCl, 100 mM Tris-HCl (pH 8.3 at 42°C), 1 mM of each deoxynucleotide triphosphate, 200 ug/ml oligo(dT)12-18, 10 mM MgCl$_2$, and 0.1 uCi $^{32}$P-dCTP/ml. These reactions are performed at 42°C for 1 hour after the addition of 3 ul of 17 units/ul AMV reverse transcriptase (Life Sciences). The reaction is terminated by the addition of 0.25 M EDTA (pH 8.0) to 20 mM. The resulting mixture is extracted once with an equal volume of phenol/chloroform (1:1) followed by one chloroform extraction. The sample is then chromatographed on a 5 ml Sepharose CL-4B column (Pharmacia) equilibrated in 10 mM Tris-HCl (pH 8.00 100 mM NaCl, 1 mM EDTA. The void volume is collected and the nucleic acids (including any RNA/cDNA hybrids) are precipitated by the addition of 2.5 volumes of ethanol.

Preferably in conjunction with the above procedures an AHF exon oligonucleotide segment is also used in place of oligo DT to prime the reverse transcription, as described in Ullrich et al., Nature, 303:821 (1983).

The RNA-cDNA hybrids are dissolved in 35 ul of deionized H$_2$O, made 100 mM potassium cacodylate (pH 6.8), 100 uM dCTP, 1 mM 2-mercaptoethanol, 1 mM cobalt chloride and enzymatically "tailed" by the addition of 10 units of deoxynucleotidyl terminal transferase (pH Biochemicals) and incubating the reaction for 30 seconds at 37°C. The reaction is terminated by adding 0.25 M EDTA to 10 mM. Tris-HCl (pH 8.0) is added to 300 mM and the sample extracted once with an equal volume of phenol-chloroform (1:1) and then with an equal volume of chloroform. Nucleic acids are precipitated from this product by the addition of 2.5 volumes of ethanol.

The dC tailed hybrid molecules are then annealed with 170 ug/ml oligo(dG)14-18 cellulose in 10 mM KCl, 1 mM EDTA for 10 minutes at 43°C and then an additional 10 minutes at 23°C. This reaction product is then diluted to 100 ul containing 100 mM ammonium sulfate, 1 mM 2-mercaptoethanol, 100 mM MgCl$_2$,

100 ug/ml bovine serum albumin (Sigma, Cohn fraction V), and 100 uM nicotinamide adenine dinucleotide. Second strand cDNA synthesis is initiated by the addition of 1 unit RNase H (P-L Biochemicals), 1 unit of E. coli DNA ligase, and 10 units of DNA polymerase I and incubated at 16°C for 12 hours.

The sample is then chromatographed over Sepharose CL-4B as described above. Double stranded DNA is ethanol precipitated and dC tailed as described for the RNA-cDNA hybrid tailing.

## 2. Screening for Human AHF DNA

dC tailed double stranded cDNA obtained as described above is annealed with an equimolar amount of dG tailed pBR322 (New England Nuclear) in 10 mM Tris-HCl (pH 8.0), 1 mM EDTA, 100 mM NaCl at 37°C for 2 hours. The annealed chimeric molecules are then frozen at -20°C until use in bacterial transformation.

Bacterial transformation is done using the MC1061 strain of E. coli. Cells (50 ml) are grown to an optical density of 0.25 at 600 nm. Cells are concentrated by centrifugation at 2,500 rpm for 12 minutes, washed in 10 ml of sterile 100 mM CaCl₂, and again pelleted by centrifugation as described above. Cells are resuspended in 2 ml of sterile 100 mM CaCl₂ and kept at 4°C for 12 hours. The annealed chimeric molecules are incubated at a ratio of 5 ng of double stranded cDNA per 200 ul competent cells at 4°C for 30 minutes. The bacteria are then subjected to a two minute heat pulse of 42°C. 1.0 ml of L-broth is then added and the cells incubated for 1 hour at 37°C. Cells are then plated onto LB-agar plates containing 5 ug/ml tetracycline.

Human AHF clones are identified using the colony hybridization procedure of Grunstein and Hogness PNAS U.S.A. 72:3961 (1975). The cDNA library is plated onto a nitrocellulose filter (Schleicher and Schuell) overlaying L-broth/ 5 ug/ml tetracycline agar plates. Colonies are grown overnight at 37°C and then the filter placed on sterile Whatman 3 M paper. A pre-moistened nitrocellulose filter is then pressed against the master filter and the filters keyed using an 18 gauge needle. The replica filter is then grown on LB-tetracycline plates at 37°C until the colonies reached a diameter of 1-2 mm. The filters are then transferred to LB plates containing 150 ug/ml chloramphenicol and incubated at 37°C for 16-24 hours.

Filters are then removed and placed onto Whatman 3 M paper saturated with 0.5 M NaOH for 5 minutes at room temperature. Filters are then neutralized by placing onto Whatman 3 M saturated with 1 M Tris-HCl (pH 7.5), 1.5 M NaCl, and then Whatman 3 M saturated with 2x standard saline citrate (SSC). SSC (1x) is 0.15 M NaCl, 0.015 M sodium citrate.

Filters are air dried and baked in vacuo at 80°C for 2 hours. Prehybridization of filters is done at 65°C for 30 minutes in 10 mM Tris-HCl (pH 8.0), 1 mM EDTA, 0.1% SDS followed by 30 minutes in 7x SSC, 5x Denhardt's (1x Denhardt's is 0.02% polyvinylpyrrolidine, 0.02% ficoll, 0.02% bovine serum albumin), 100 ug/ml denatured salmon sperm DNA, and 0.1% SDS. $^{32}$P-labelled human exon DNA, prepared as described above, are added to $10^6$ cpm/ml and the hybridization performed 12-16 hours at 37°C. Filters are then washed in several changes of 7x SSC, 0.1% SDS for 1-2 hours at 37°C. Filters are then air-dried and subjected to autoradiography with Kodak XAR film and a Dupont Lightning Plus intensifying screen.

Those colonies showing a hybridization signal above background are grown in L-broth containing 50 ug/ml tetracycline for rapid prep purification of plasmid DNA. Plasmid DNA is purified by the method of Holmes et al., Anal. Biochem., 114:193 (1981). An aliquot of this DNA is cleaved with restriction endonuclease Pst 1 and the fragments electrophoresed through 1% agarose/TBE gels and blotted according to the procedure of E. Southern, J. Mol, Biol. 98:503 (1975), Methods Enzymol. 69:152 (1980). The nitrocellulose filters are hybridized with radiolabeled human AHF exon DNA as described for colony hybridization. Those plasmids which contained Pst I inserts hybridizing to the AHF exon DNA are used for DNA sequencing analysis.

For sequencing, plasmid DNA, (purified from 0.75 ml of culture by the procedure of Holmes, et al., supra), is digested to completion with the restriction endonuclease. The Sau 3 A I resulting DNA segment, identified as 34-S1, has a DNA sequence which is depicted in Figure 4. The DNA is ethanol precipitated after extraction with phenol-chloroform and redissolved in 10 ul TE (10 mM Tris-HCl pH 8.0, 1 mM EDTA). 5 ul of the DNA solution is ligated with 20 ng of Bam H 1 cleaved M13 mp 9 replicative form DNA in 100 ul of 50 mM Tris-HCl (pH 7.4), 10 mM MgCl₂, 10 mM dithiothreitol, 1 mM ATP and an excess of T4 DNA ligase. Ligations are done at 15°C for 2-4 hours.

5 ul of the ligation reactions are used to transform 200 ul of E. coli strain JM101/TG1 as described above. Recombinants are identified as white plaques when grown on LB-agar plates containing X-gal as an indication for beta-galactosidase activity as described by Davies, et al., J. Mol. Biol. 36:413 (1968).

Recombinant phage harboring sequences hybridizing to the human exon are identified by the procedure of Benton, et al., Science 196:180 (1977), using radioactively labelled human AHF exon as a probe. Plaques showing a hybridization signal are picked and grown in 1.5 ml cultures of L-broth. Single-stranded phage DNA prepared from these cultures is used as template in oligonucleotide-primed DNA synthesis reactions. Sequencing is done using the dideoxy chain-termination procedure, see, e.g., Sanger et al., PNAS U.S.A. 74:5463 (1977).

Human AHF recombinants are identified by comparing their nucleotide sequence with that which is known from the human exon sequence of human AHF.

### 3. Porcine AHF mRNA

Human AHF recombinants are used to screen a porcine tissue library constructed exactly as described for the human tissue cDNA library. Prospective porcine AHF recombinant DNA clones are identified by the Grunstein-Hogness procedure using the porcine $^{32}$P labeled exon fragment as a probe as described above. The probe is the porcine AHF exon segment labelled with $^{32}$P by nick-translation as described by Rigby et al., J. Mol. Biol., 113-237 (1977).

Colonies exhibiting hybridization signals are grown for rapid prep plasmid purification purposes as described above. Plasmid DNA is cleaved with the restriction endonuclease Pst I, electrophoresed through 1% agarose/TBE gels, and blotted according to the procedure of E. Southern (1975). The blots are hybridized with nick-translated porcine AHF recombinant DNA labelled with $^{32}$P.

Full-length clones may be constructed in a conventional manner, such as by ligation of DNA fragments from overlapping clones at restriction enzyme sites common to both clones as is well known in the art ("gene walking").

### 4. Identifying full-length clones from steps 2 or 3

The distance between the 5' end of an existing clone and the 5' end of the mRNA can be analyzed by the primer-extension technique described in Agarwal et al., "J.B.C." 256(2):1023-1028 (1981) utilizing an oligonucleotide primer whose sequence comes from the 5' (amino-terminal) region of the existing AHF clone. If gels developed using this procedure show more than one transcript one should consider the most intense band as representing the full mRNA transcript.

There are, however, many mRNAs which contain a long region of 5' untranslated sequence. Thus, expression of human AHF DNA may not be contingent upon the acquisition of a complete cDNA clone. For example, a clone may be obtained which by DNA sequencing analysis demonstrates the existence of a methionine codon followed by a sequence which is analogous to or identical to a known eucaryotic protein secretion signal and which is in frame with the remaining codons. Transformation and expression should be conducted for a clone which contains the met-secretion signal sequence, which is of the expected size and which contains a poly (T) 3' terminus.

### 5. Alternative Procedure

As an alternative to the methods in Sections 1-3 described above, it is preferred that the cDNA clones for porcine or human AHF be identified using a bacteriophage vector in the following manner.

mRNA from human fetal liver tissue responsible for AHF synthesis was prepared by the guanidine hydrochloride method as described by Cox and modified by Chirgwin et al. as set forth above in Example 5, Section 1. First strand cDNA was synthesized from 10 ug of polyA$^+$ fetal liver RNA by the procedure described in Example 6, Section 1, supra. Specifically, 10 ug of this RNA was denatured at room temperature in 10 ul of 10 mM methylmercuryhydroxide. 140 mM 2-mercaptoethanol was added to inactivate the methylmercuryhydroxide. The RNA was then diluted to 50 ul containing 140 mM KCl, 100 mM Tris-HCl (pH 8.3 at 42 °C), 1 mM of each deoxynucleotide triphosphate, 200 ug/ml oligo(dT)12-18, 10 mM MgCl$_2$, and 0.1 uCi -dCTP/ml. These reactions were performed at 42°C for 1 hour after the addition of 3 ul of 17 units/ul AMV reverse transcriptase (Life Sciences).

For the first strand synthesis of a primer-extended library, 200 picomoles of a unique complimentary 38mer was included in the CH$_3$HgOH denaturation step, and after 10 minutes at 23°C, the reaction was made 140mM beta-mercaptoethanol, 0.7M KCl, 1mM EDTA, 20mM Tris-HCl (pH 8.3 at 42°C), 1 unit/ul of RNasin (Biotec) and incubated at 50°C for 2 minutes and at 42°C for 2 minutes. This was then diluted to 50 ul containing 140 mM KCl, 100 mM Tris-HCl (pH 8.3 at 42°C), 1 mM of each deoxynucleotide triphosphate, 10 mM MgCl$_2$, and 0.1 uCi $^{32}$P-dCTP/ul. The reaction was incubated at 42 °C for 1 hour after the addition of 3 ul of 17u/ul AMV reverse transcriptase.

After first strand synthesis the reactions were diluted to 150ul containing 10mM MgCl$_2$, 50mM Tris pH 7.4, 5mM 2-mercaptoethanol, 7.5mM NH$_4$SO$_4$, 250 uM of each deoxynucleotide triphosphate, and second strand synthesis initiated by the addition of 1 unit of RNase H (E. coli) and 45 units of DNA polymerase I. Reactions were incubated at 16°C for 8 hours and then terminated by the addition of EDTA to 20mM and brought to a final volume of 200ul with $H_2O$. EcoRI methylation was then performed by addition of S-adenosyl methionine to 50uM and 40 units of EcoRI methylase. These reactions were incubated for 1 hour at 37°C, terminated by phenol-chloroform extraction, and chromatographed using Sephadex G50 equilibrated in 10mM Tris-HCl (pH 8.0), 1mM EDTA, 0.1M NaCl. The void volume was pooled and precipitated by ethanol addition.

cDNA molecules were blunt-ended in 200ul containing 50mM Tris pH 8.3, 10mM $MgCl_2$, 10mM 2-mercaptoethanol, 50mM NaCl, 50uM of each deoxynucleotide triphosphate, 100ug/ml ovalbumin and 5 units of T4 polymerase. The reaction was incubated at 37°C for 30 minutes and then terminated by phenol-chloroform extraction. Nucleic acids were then precipitated by ethanol addition.

EcoRI "linkers" (Collaborative Research) were then ligated to the blunted cDNA molecules under standard conditions, Maniatis, et al., supra, at 243, in a total volume of 45ul. The reactions were terminated by the addition of EDTA to 15mM and extracted with phenol-chloroform. Nucleic acids were precipitated by ethanol and pelleted by centrifugation. The linkered cDNA was redissolved in 200ul of 100uM Tris-HCl (pH 7.2), 5mM $MgCl_2$, 50mM NaCl and digested with 300 units of EcoRI for 2 hours at 37°C. The reaction was then extracted with phenol-chloroform and chromatographed over Sepharose CL-4B equilibrated with 10mM Tris (pH 8.0), 1mM EDTA, 0.1M NaCl. cDNA in the void volume was collected, precipitated by ethanol and pelleted by centrifugation.

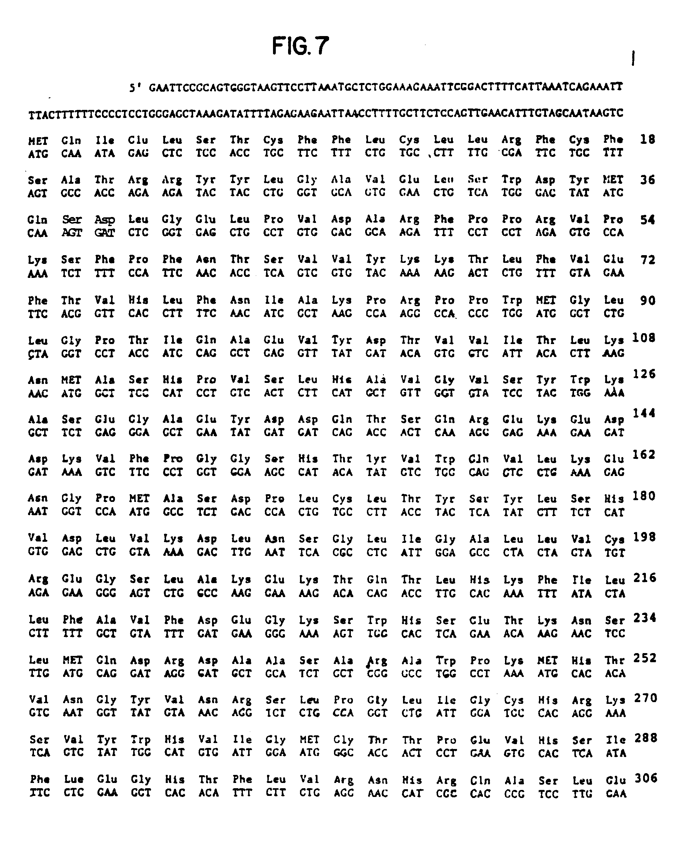

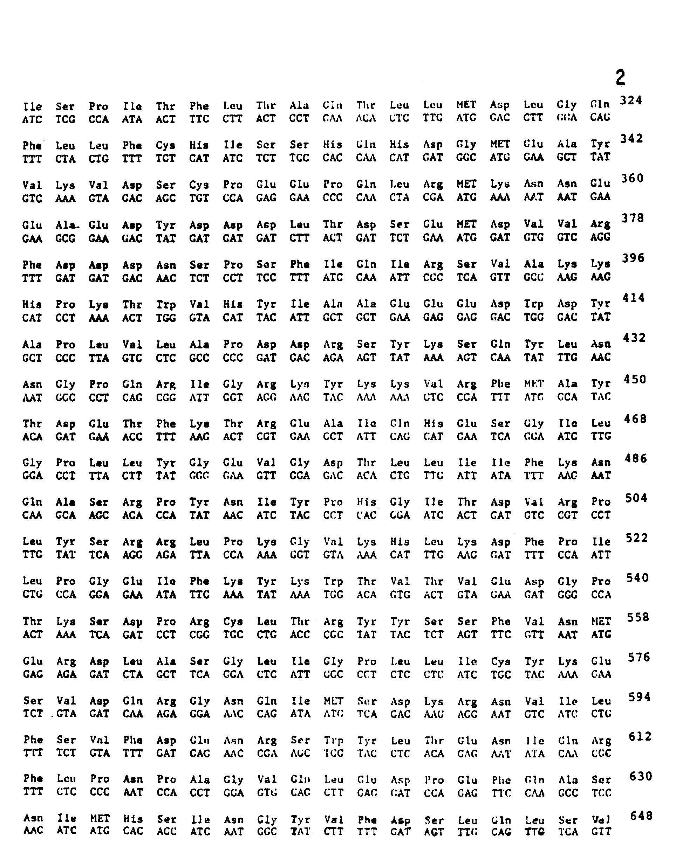

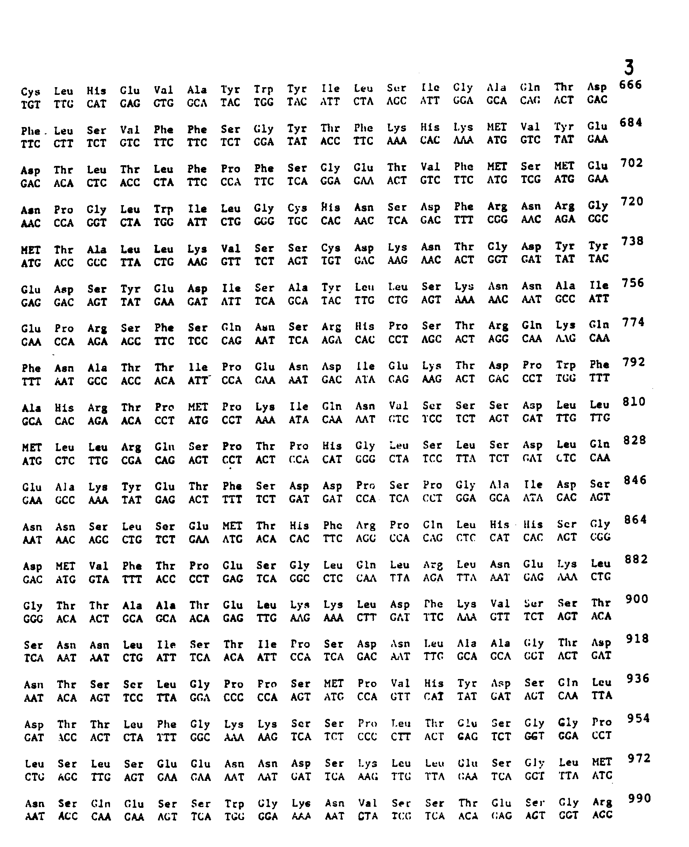

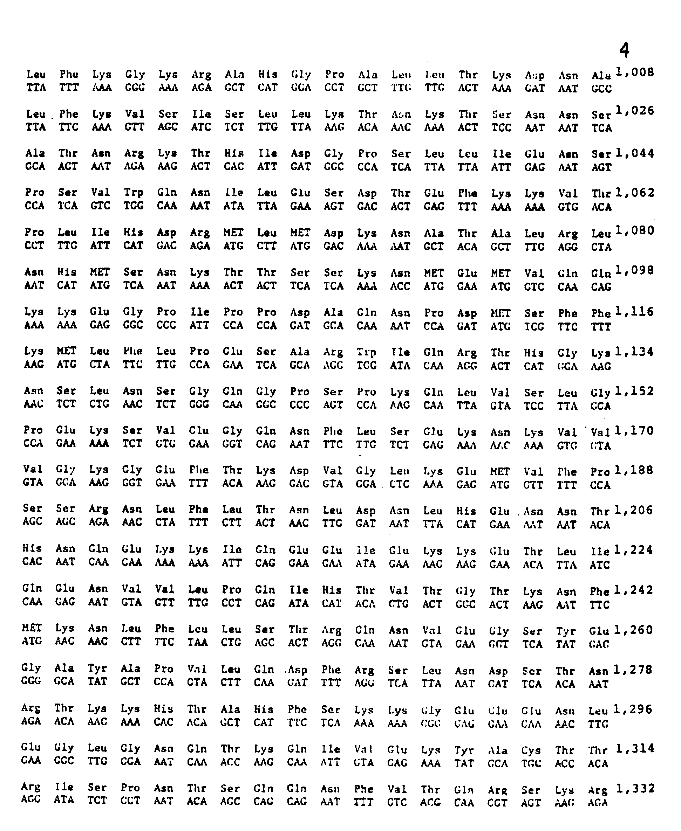

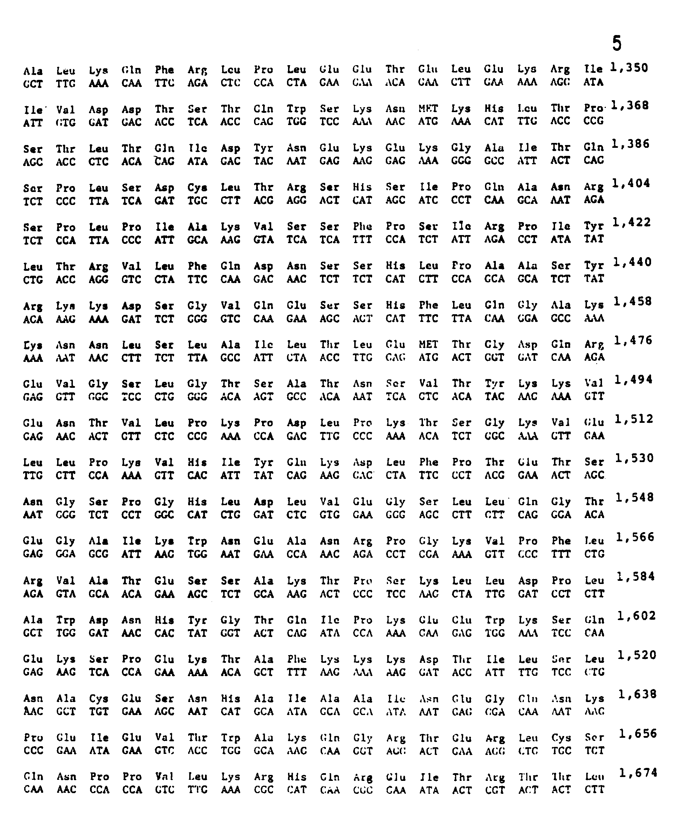

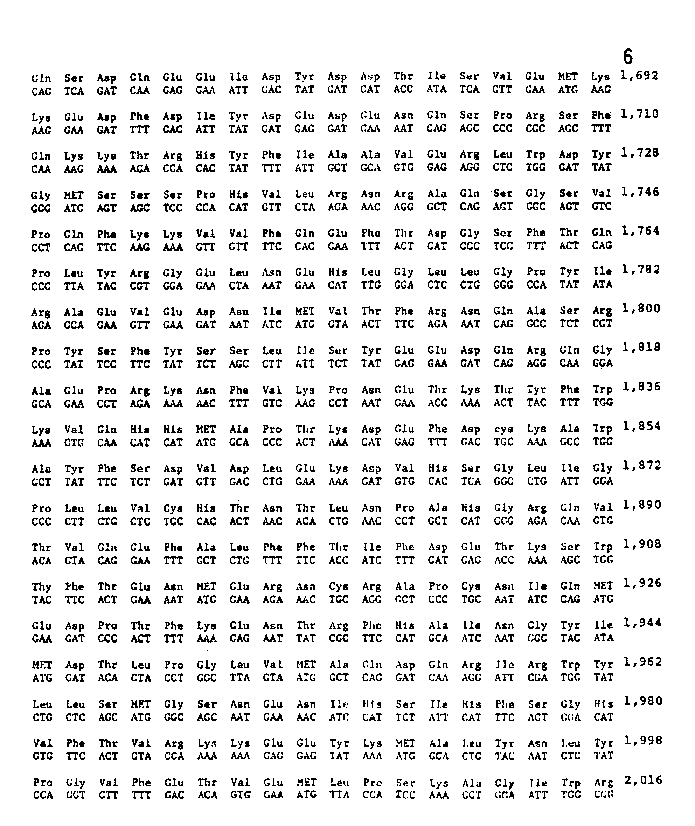

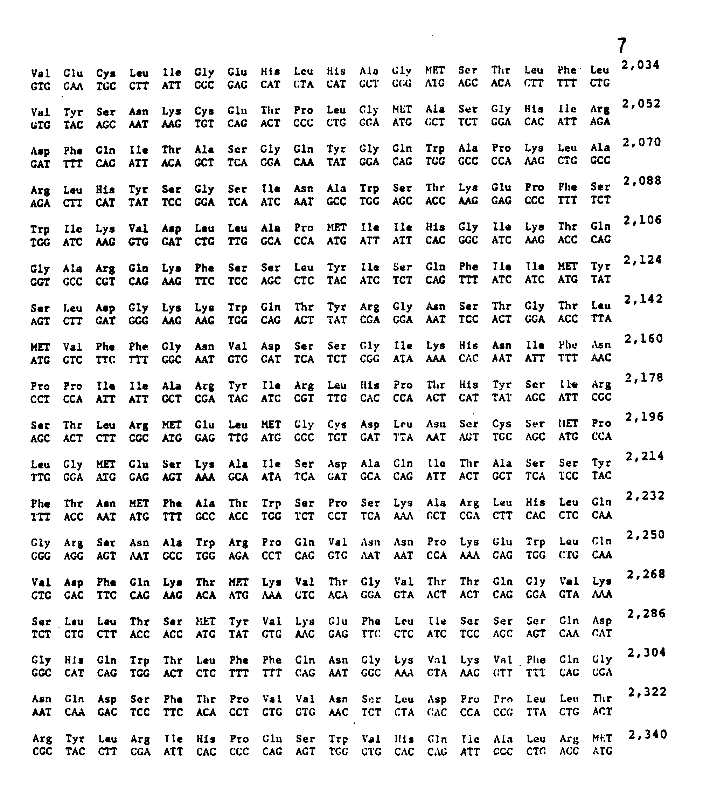

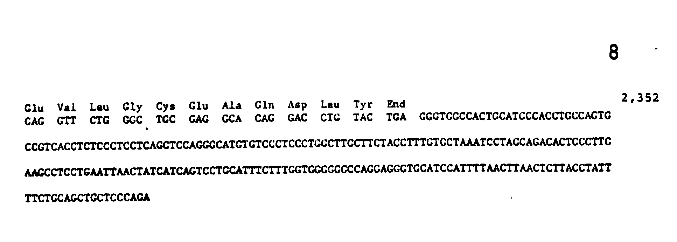

cDNA was redissolved in 10mM Tris-HCl (pH 8.0), 1mM EDTA and ligated to EcoRI cleaved, phosphatased lambda Charon 21A DNA at various ratios of cDNA to vector DNA using standard ligation conditions. Ligated DNA was packaged and titered using established procedures, Maniatis, et al., supra, at 64, 256. The library was plated and screened using $^{32}$P-labelled human exon DNA under conditions described in Benton and Davis, supra. Overlapping clones which spanned approximately 10,000 base pairs were obtained and a substantial portion thereof was sequenced to reveal one long open reading frame end human AHF. The recombinant DNA nucleotide sequence obtained therefrom coding for human AHF is shown in Fig. 7 along with the deduced amino acid sequence for human AHF. The overlaping clones were assembled into vector pSP64 (Promega Biotec) using conventional techniques well known in the art, i.e. by ligation of DNA fragments from overlaping clones at restriction enzyme sites common to both clones. A pSP64 recombinant clone containing the nucleotide sequence depicted in Figure 7, designated as pSP64-VIII, is on deposit at the American Type Culture Collection under Accession Number ATCC 39812.

EXAMPLE 7

Expression of human or porcine AHF

This example contemplates expression of AHF using the full length clone obtained by the method of Example 6 in a cotransformation system.

1. Preparation of Transformation Vector

A direct method for obtaining the AHF transformation vector is described below. The pCVSVL plasmid is partially digested with Pst I, the digest separated on gel electrophoresis. After visualization, the linear DNA fragment band corresponding to full length plasmid is isolated as pCVSVL-Bl.

The cDNA for AHF is rescued from the Example 6 cloning vectors by partial digest with Pst I. The partial digest is separated on gel electrophoresis, and the band corresponding to the molecular weight of the full length cDNA is isolated. pCVSVL-Bl is annealed with this DNA fragment, ligated with T4 ligase at 15°C, transfected into E. coli strain HB101 and transformants selected for tetracycline resistance. The selected E. coli transformants are grown in the presence of tetracycline. Plasmid pCVSVL-Bla is recovered in conventional fashion. Proper orientation of the cDNA in the plasmid may be determined in conventional fashion by assymetric digestion with an appropriate endonuclease(s).

2. Cotransformation; Selection and Amplification

Plasmid pCVSVL-Ala or pCVSVL-Bla and pAdD26SVpA#3 (Kaufman et al., op. cit.) are mixed together (50 ug HP and 0.5 ug pAdD26SVpA #3) and precipitated by the addition of NaOAc pH 4.5 to 0.3M and 2.5 vols. of ethanol. Precipitated DNA is allowed to air dry, is resuspended in 2X HEBSS (0.5ml) and mixed vigorously with 0.25 M $CaCl_2$ (.5ml) as described (Kaufman et al., op. cit). The calcium-phosphate-DNA precipitate is allowed to sit 30' at room temperature and applied to CHO DUKX-B1 cells (Chasin and Urlaub, 1980, available from Columbia University). The growth and maintenance of these cells has been described (Kaufman et al., op. cit, Chasin and Urlaub 1980).

The DUKX-B1 cells are subcultured at $5\times10^5$/10cm dish 24 hr. prior to transfection. After 30 minutes incubation at room temperature, 5ml of a media with 10% fetal calf serum is applied and the cells are incubated at 37°C for 4.5 hr. The media is then removed from the monolayer, 2ml of a-media with 10% fetal calf serum, 10ug/ml of thymidine, adenosine, and deoxyadenosine, and penicillin and streptomycin. Two days later the cells are subcultured 1:15 into a-media with 10% dialyzed fetal calf serum, and penicillin and streptomycin, but lacking nucleosides. Cells are then fed again with the same media after 4-5 days.

Colonies appear 10-12 days after subculturing into selective media. Methotrexate (MTX) selection and detection of AHF gene and selection gene amplification are conducted in accordance with Axel et al, U.S. Patent 4,399,216, or Kaufman et al., op. cit.

AHF yields may be improved by cotransforming the permanent cell line EA.hy 926 (Edgell et al., "Proc. Natl. Acad. Sci" 80:3734-3737 (1983) in place of DUKX-B1 cells. In this case the cotransforming selection gene should be the dominant DHFR gene disclosed by Haber et al., "Somatic Cell Genetics" 4:499-508 (1982). Otherwise, the cotransformation and culture will be substantially as set forth elsewhere herein.

### 3. Production of AHF

AHF-producing CHO transformants selected in Section 2 are maintained in seed culture using standard techniques. The culture is scaled up to 10 liters by cell culture in conventional media. This medium need not contain MTX and will not contain nucleosides so as to exclude selection of gene revertants.

The culture supernatant is monitored for clotting activity following standard assays for use with blood plasma samples (reduction in clotting time of Factor VIII-deficient plasma). The supernatant may be purified by conventional techniques such as polyethylene glycol and glycine precipitations (as are known for use in purifying AHF from blood plasma) in order to increase the sensitivity of the Factor VIII assays.

When Factor VIII activity has reached a peak the cells are separated from the culture medium by centrifugation. The Factor VIII is then recovered and purified by polyethylene glycol and glycine precipitations. Clotting activity is demonstrated in Factor VIII deficient plasma.

### 4. Alternative Procedure for Production of AHF

A full length cDNA containing the entire AHF coding region was constructed from the exon in HH-25 described above, two cDNA clones which overlapped the extreme 5' and 3' regions of that exon, and a third clone which overlapped the 3' cDNA clone and continued past the termination codon. This was constructed such that synthetic Sal I sites were placed just 5' to the initiator methionine and at a position 3' to the translational stop signal at the end of the AHF coding sequences. This allowed the placement into and excision from the polylinker Sal I site of pSP64. The Sal I fragment from this clone (pSP64-VIII) was purified and ligated to pCVSVL2. pCVSVL2 is a plasmid which is identical to the expression vector pCVSVL (Kaufman, et al., Mol. Cell Biol., 2:1304 (1982), except that it has deleted the Pst I site located 3' of the SV40 polyadenylation sequence which is accomplished by conventional procedures and that it contains a duplication of the SV40 Ava II "D" fragment upstream from the adenovirus major late promoter (MLP). pCVSVL2 was derived from pAdD26SVpA(1) (See Kaufman et al., supra) by adding XhoI linkers at each end of two SV40 Ava II "D" fragments, and inserting them into the XhoI site in pAdD26SVpA(1). The Ava II "D" fragments are both inserted such that the SV40 late promoter is in the same orientation as the Ad2 major late promoter. For plasmids pCVSVL and pAdD26SVpA(3), see Kaufman et al., supra. To insert the Sal I fragment of pSP64-VIII into pCVSVL2, the unique Pst I site in pCVSVL2 was converted to a Sal I site by the procedure described in Rothstein et al., Methods Enzym., 69:98 (1980), and the Sal I fragment excised from pSP64-VIII was inserted to yield pCVSVL2-VIII. pCVSVL2-VIII contains the correct 5' to 3' orientation which allows transcription of the AHF coding sequence from the adenovirus MLP of the vector. pCVSVL2-VIII is on deposit at the American Type Culture Collection under Accession Number 39813.

The pCVSVL2-VIII was introduced into monkey COS-7 cells (Mellon et al., Cell 27:279 (1981) using the DEAE dextran transfection protocol described by Sampayrac and Danna, PNAS U.S.A. 78:7575 (1981). The cells were cultured in serum-free medium which was assayed for AHF activity 3 days after transfection.

Factor VIII:C activity was determined by the chromogenic substrate assay, described by Didisheim, Science 129:389 (1959), which confirmed that human Factor VIII:C expression had been achieved at a level of about 0.05 units/ml.

**Claims**

**Claims for the following Contracting States : BE, CH, DE, FR, GB, LI, LU, NL, SE**

1. An isolated recombinant vector containing DNA coding for human factor VIII:C, comprising a polydeoxyribonucleotide having the sequence:

$$5' \ CGC \ AGC \ TTT \ CAG \ AAG \ AAA \ ACA \ CGA \ CAC$$

$$TAT \ TTT \ ATT \ GCT \ GCA \ GTG \ GAG \ AGG \ 3'$$

2. The recombinant vector DNA of claim 1, wherein said DNA is human genomic DNA.

3. The vector of claim 11 wherein the DNA coding for human factor VIII:C is linked directly or indirectly to DNA From a non-human source.

4. A non-human recombinant expression vector for human factor VIII:C comprising a DNA segment having the sequence:

$$5 \ 'CGC \ AGC \ TTT \ CAG \ AAG \ AAA \ ACA \ CGA \ CAC$$

$$TAT \ TTT \ ATT \ GCT \ GCA \ GTG \ GAG \ AGG \ 3'$$

operatively linked to a heterologous regulatory control sequence.

5. A transformed non-human mammalian cell line containing the expression vector of claim 4.

6. A screening agent for identifying deoxyribonucleotide sequences and ribonucleotide sequences which encode for at least a portion of the human gene factor VIII:C, consisting essentially of a deoxyribonucleotide having the following sequence or its inverse complement:

$$5' \ TTT \ CAG \ AAG \ AGA \ ACC \ CGA \ CAC \ TAT \ TTC$$

$$ATT \ GCT \ GCG \ GTG \ GAG \ CAG \ CTC \ TGG \ GAT$$

$$TAC \ GGG \ ATG \ AGC \ GAA \ TCC \ CCC \ CGG \ GCG$$

$$CTA \ AGA \ AAC \ AGG \ 3'.$$

7. A method of producing a protein having human factor VIII:C procoagulant activity, comprising the steps of:
   (1) culturing an isolated cell transformed with double stranded DNA which DNA
       (a) has been operatively linked to a heterologous regulatory control sequence,
       (b) hybridizes under stringent conditions to the following nucleotide sequence:

$$5' \ GAC \ ATT \ TAT \ GAT \ GAG \ GAT \ GAA$$

$$ATT \ CAG \ AGC \ CCC \ CGC \ AGC \ TTT$$

$$CAG \ AAG \ AAA \ ACA \ CGA \ CAC \ TAT$$

$$TTT \ ATT \ GCT \ GCA \ GTG \ GAG \ AGG \ 3'$$

and
   (c) encodes a protein which upon expression exhibits human factor VIII:C procoagulant activity, and

(2) recovering said protein from the culture.

8. A transformed non-human mammalian cell containing a gene encoding human factor VIII:C comprising a polydeoxyribonucleotide having the sequence:

$$5 \text{ 'CGC AGC TTT CAG AAG AAA ACA CGA CAC}$$
$$\text{TAT TTT ATT GCT GCA GTG GAG AGG 3'}$$

in operable association with a heterologous regulatory control sequence in an expression vector therefor.

**Claims for the following Contracting State : AT**

1. Use of a DNA sequence coding for human factor VIII:C, comprising a polydeoxyribonucleotide having the sequence

$$5' \text{ GAC ATT TAT GAT GAG GAT GAA}$$
$$\text{ATT CAG AGC CCC CGC AGC TTT}$$
$$\text{CAG AAG AAA ACA CGA CAC TAT}$$
$$\text{TTT ATT GCT GCA GTG GAG AGG 3'}$$

for producing an isolated recombinant vector containing this DNA sequence.

2. Use of the DNA sequence of claim 1, wherein the DNA is human genomic DNA.

3. Use of the DNA sequence of claim 1 for producing a recombinant vector, wherein the DNA sequence is linked directly or indirectly to DNA from a non-human source.

4. Use of the DNA sequence of claim 1 for producing a non-human recombinant expression vector, wherein the DNA sequence is operatively linked to a heterologous regulatory control sequence.

5. A transformed non-human mammalian cell line containing the expression vector of claim 4.

6. Use of a DNA sequence consisting essentially of a deoxyribonucleotide having the following sequence or its inverse complement

$$5' \text{ TTT CAG AAG AGA ACC CGA CAC TAT TTC}$$
$$\text{ATT GCT GCG GTG GAG CAG CTC TGG GAT}$$
$$\text{TAC GGG ATG AGC GAA TCC CCC CGG GCG}$$
$$\text{CTA AGA AAC AGG 3'}$$

as a screening agent for identifying deoxyribonucleotide sequences and ribonucleotide sequences which encode for at least a portion of the human gene factor VIII:C.

7. A method of producing a protein having human factor VIII:C procoagulant activity, comprising the steps of:
   (1) culturing an isolated cell transformed with double stranded DNA which DNA
       (a) has been operatively linked to a heterologous regulatory control sequence,
       (b) hybridizes under stringent conditions to the following nucleotide sequence:

```
5' GAC ATT TAT GAT GAG GAT GAA
   ATT CAG AGC CCC CGC AGC TTT
   CAG AAG AAA ACA CGA CAC TAT
   TTT ATT GCT GCA GTG GAG AGG 3'
```

and

(c) encodes a protein which upon expression exhibits human factor VIII:C procoagulant activity, and

(2) recovering said protein from the culture.

8. A transformed non-human mammalian cell containing a gene encoding human factor VIII:C comprising a polydeoxyribonucleotide having the sequence:

```
5' CGC AGC TTT CAG AAG AAA ACA CGA CAC
   TAT TTT ATT GCT GCA GTG GAG AGG 3'
```

in operable association with a heterologous regulatory control sequende in an expression vector therefore.

**Revendications**
**Revendications pour les Etats Contractants suivants : BE, CH, DE, FR, GB, LI, LU, NL, SE**

1. Vecteur recombinant isolé contenant l'ADN codant pour le facteur humain VIII:C, comprenant un polydésoxyribonucléotide ayant la séquence :

```
5' CGC AGC TTT CAG AAG AAA ACA CGA CAC
   TAT TTT ATT GCT GCA GTG GAG AGG 3'
```

2. Vecteur recombinant contenant l'ADN selon la revendication 1, dans lequel l'ADN est un ADN génomique humain.

3. Vecteur selon la revendication 1, dans lequel l'ADN codant pour le facteur humain VIII:C est lié directement ou indirectement à l'ADN provenant d'une source non humaine.

4. Vecteur d'expression recombinante non humaine pour le facteur humain VIII:C comprenant un segment d'ADN ayant la séquence :

```
5 'CGC AGC TTT CAG AAG AAA ACA CGA CAC
   TAT TTT ATT GCT GCA GTG GAG AGG 3'
```

relié de façon opérante à une séquence de commande régulatrice hétérologue.

5. Lignée de cellules mammifères non humaines transformées contenant le vecteur d'expression de la revendication 4.

6. Agent de criblage pour l'identification de séquences de désoxyribonucléotide et de séquences de ribonucléotide qui codent pour au moins une portion du facteur de gène humain VIII:C consistant essentiellement en un désoxyribonucléotide ayant la séquence suivante ou son complément inverse :

```
5' TTT CAG AAG AGA ACC CGA CAC TAT TTC
   ATT GCT GCG GTG GAG CAG CTC TGG GAT
   TAC GGG ATG AGC GAA TCC CCC CGG GCG
   CTA AGA AAC AGG 3'.
```

7. Procédé destiné à produire une protéine ayant une activité procoagulante de facteur humain VIII:C, comprenant les étapes consistant à :
   (1) mettre en culture une cellule isolée transformée par un ADN bicaténaire, lequel ADN
      (a) est lié de façon opérante à une séquence de commande régulatrice hétérologue,
      (b) s'hybride dans des conditions rigoureuses à la séquence nucléotide suivante :

```
5' GAC ATT TAT GAT GAG GAT GAA
   ATT CAG AGC CCC CGC AGC TTT
   CAG AAG AAA ACA CGA CAC TAT
   TTT ATT GCT GCA GTG GAG AGG  3'
```

      et
      (c) code une protéine, laquelle sur expression présente une activité procoagulante de facteur humain VIII:C, et
   (2) récupérer la protéine de la culture.

8. Cellule mammifère non humaine transformée contenant un gène codant le facteur humain VIII:C comprenant un polydésoxyribonucléotide ayant la séquence :

```
5 'CGC AGC TTT CAG AAG AAA ACA CGA CAC
   TAT TTT ATT GCT GCA GTG GAG AGG 3'
```

en association utilisable avec une séquence de commande régulatrice hétérologue dans un vecteur d'expression pour celle-ci.

**Revendications pour l'Etat contractant suivant : AT**

1. Utilisation d'une séquence d'ADN codant pour le facteur humain VIII:C, comprenant un polydésoxyribonucléotide ayant la séquence :

```
5' GAC ATT TAT GAT GAG GAT GAA
   ATT CAG AGC CCC CGC AGC TTT
   CAG AAG AAA ACA CGA CAC TAT
   TTT ATT GCT GCA GTG GAG AGG 3'
```

pour produire un vecteur recombinant isolé contenant cette séquence d'ADN.

2. Utilisation de la séquence d'ADN de la revendication 1, dans laquelle l'ADN est un ADN génomique humain.

3. Utilisation de la séquence d'ADN de la revendication 1 pour produire un vecteur recombinant, dans lequel la séquence d'ADN est liée directement ou indirectement à l'ADN provenant d'une source non humaine.

4. Utilisation de la séquence d'ADN selon la revendication 1 pour produire un vecteur d'expression recombinante non humaine, dans lequel la séquence d'ADN est liée de façon opérante à une séquence de commande régulatrice hétérologue.

5. Lignée de cellules mammifères non humaines transformées contenant le vecteur d'expression de la revendication 4.

6. Utilisation d'une séquence d'ADN consistant essentiellement en un désoxyribonucléotide ayant la séquence suivante ou son complément inverse :

```
5' TTT CAG AAG AGA ACC CGA CAC TAT TTC
   ATT GCT GCG GTG GAG CAG CTC TGG GAT
   TAC GGG ATG AGC GAA TCC CCC CGG GCG
   CTA AGA AAC AGG 3'
```

en tant qu'agent de criblage pour identifier les séquences de désoxyribonucléotide et les séquences de ribonucléotide qui codent pour au moins une portion du facteur de gène humain VIII:C.

7. Procédé pour produire une protéine ayant une activité procoagulante de facteur humain VIII:C, comprenant les étapes consistant à :
(1) mettre en culture une cellule isolée transformée par un ADN bicaténaire, lequel ADN
(a) est lié de façon opérante à une séquence de commande régulatrice hétérologue,
(b) s'hybride dans des conditions rigoureuses sur la séquence nucléotide suivante :

```
5' GAC ATT TAT GAT GAG GAT GAA
   ATT CAG AGC CCC CGC AGC TTT
   CAG AAG AAA ACA CGA CAC TAT
   TTT ATT GCT GCA GTG GAG AGG 3'
```

et
(c) code une protéine, laquelle sur expression présente un activité procoagulante de facteur humain VIII:C, et
(2) récupérer la protéine de la culture.

8. Cellule mammifère non humaine transformée contenant un gène codant le facteur humain VIII:C comprenant un polydésoxyribonucléotide ayant la séquence :

```
5' CGC AGC TTT CAG AAG AAA ACA CGA CAC
   TAT TTT ATT GCT GCA GTG GAG AGG 3'
```

en association utilisable avec une séquence de commande régulatrice hétérologue dans un vecteur d'expression pour celle-ci.

**Patentansprüche**
**Patentansprüche für folgende Vertragsstaaten : BE, CH, DE, FR, GB, LI, LU, NL, SE**

25

1. Isolierter rekombinanter Vektor, enthaltend DNA, die für menschlichen Faktor VIII:C codiert, der ein Polydesoxyribonukleotid mit der Sequenz umfaßt:

```
5' CGC AGC TTT CAG AAG AAA ACA CGA CAC
   TAT TTT ATT GCT GCA GTG GAG AGG 3'
```

2. Die DNA des rekombinanten Vektors nach Anspruch 1, worin die DNA menschliche genomische DNA ist.

3. Vektor nach Anspruch 1, worin die für menschlichen Faktor VIII:C codierende DNA mittelbar oder indirekt mit DNA nicht-menschlichen Ursprungs gekoppelt ist.

4. Nicht-menschlicher rekombinanter Expressionsvektor für menschlichen Faktor VIII:C, der ein DNA-Segment mit der Sequenz:

```
5' CGC AGC TTT CAG AAG AAA ACA CGA CAC
   TAT TTT ATT GCT GCA GTG GAG AGG 3'
```

umfaßt, das operativ mit einer heterologen regulatorischen Kontrollsequenz gekoppelt ist.

5. Transformierte nicht-menschliche Säugerzellinie, enthaltend den Expressionsvektor nach Anspruch 4.

6. Testmittel zur Identifikation von Desoxyribonukleotidsequenzen und Ribonukleotidsequenzen, die mindestens für einen Teil des menschlichen Gens für Faktor VIII:C codieren, das im wesentlichen aus einem Desoxyribonukleotid mit der folgenden Sequenz oder ihrem inversen Komplement besteht:

```
5' TTT CAG AAG AGA ACC CGA CAC TAT TTC
   ATT GCT GCG GTG GAG CAG CTC TGG GAT
   TAC GGG ATG AGC GAA TCC CCC CGG GCG
   CTA AGA AAC AGG 3'.
```

7. Verfahren zur Herstellung eines Proteins mit der Gerinnungsaktivität des menschlichen Faktors VIII:C, das die Schritte umfaßt:
   (1) Kultivierung einer isolierten, mit doppelsträngiger DNA transformierten Zelle, worin die DNA
       (a) operativ mit einer heterologen regulatorischen Kontrolsequenz gekoppelt wurde,
       (b) unter strikten Bedingungen an die folgende Nukleotidsequenz hybridisiert:

```
5' GAC ATT TAT GAT GAG GAT GAA
   ATT CAG AGC CCC CGC AGC TTT
   CAG AAG AAA ACA CGA CAC TAT
   TTT ATT GCT GCA GTG GAG AGG 3'
```

und
       (c) für ein Protein codiert, das nach Expression Gerinnungsaktivität des menschlichen Faktors VIII:C zeigt, und
   (2) Gewinnung des Proteins aus der Kultur.

**8.** Transformierte nicht-menschliche Säugerzelle, die ein für menschlichen Faktor VIII:C codierendes Gen enthält, das ein Polydesoxyribonukleotid mit der Sequenz:

$$5' \text{ CGC AGC TTT CAG AAG AAA ACA CGA CAC}$$
$$\text{TAT TTT ATT GCT GCA GTG GAG AGG } 3'$$

in operabler Verbindung mit einer heterologen regulatorischen Kontrollsequenz in einem Expressionsvektor herfür umfaßt.

**Patentansprüche für folgenden Vertragsstaat : AT**

**1.** Verwendung einer DNA-Sequenz, die für menschlichen Faktor VIII:C codiert und ein Polydesoxyribonukleotid mit der Sequenz

$$5' \text{ GAC ATT TAT GAT GAG GAT GAA}$$
$$\text{ATT CAG AGC CCC CGC AGC TTT}$$
$$\text{CAG AAG AAA ACA CGA CAC TAT}$$
$$\text{TTT ATT GCT GCA GTG GAG AGG } 3'$$

umfaßt, zur Produktion eines isolierten rekombinanten Vektors, der diese DNA-Sequenz enthält.

**2.** Verwendung der DNA-Sequenz nach Anspruch 1, worin die DNA menschliche genomische DNA ist.

**3.** Verwendung der DNA-Sequenz nach Anspruch 1 zur Produktion eines rekombinanten Vektors, worin die DNA-Sequenz mittelbar oder indirekt mit DNA nichtmenschlichen Ursprungs gekoppelt ist.

**4.** Verwendung der DNA-Sequenz nach Anspruch 1 zur Produktion eines nichtmenschlichen rekombinanten Expressionsvektors, worin die DNA-Sequenz operativ mit einer heterologen regulatorischen Kontrollsequenz gekoppelt ist.

**5.** Transofrmierte nicht-menschliche Säugerzellinie, enthaltend den Expressionsvektor nach Anspruch 4.

**6.** Verwendung einer DNA-Sequenz, die im wesentlichen aus einem Desoxyribonukleotid mit der folgenden Sequenz oder ihrem inversen Komplement besteht:

$$5' \text{ TTT CAG AAG AGA ACC CGA CAC TAT TTC}$$
$$\text{ATT GCT GCG GTG GAG CAG CTC TGG GAT}$$
$$\text{TAC GGG ATG AGC GAA TCC CCC CGG GCG}$$
$$\text{CTA AGA AAC AGG } 3'$$

als Testmittel zur Identifikation von Desoxyribonukleotidsequenzen und Ribonukleotidsequenzen, die mindestens für einen Teil des menschlichen Gens für Faktor VIII:C codieren.

**7.** Verfahren zur Herstellung eines Proteins mit der Gerinnungsaktivität des menschlichen Faktors VIII:C, das die Schritte umfaßt:
(1) Kultivierung eines isolierten, mit doppelsträngiger DNA transformierten Zelle, worin die DNA
    (a) operative mit einer heterologen regulatorischen Kontrollsequenz gekoppelt wurde,
    (b) unter strikten Bedingungen an die folgende Nukleotidsequenz hybridisiert:

```
5' GAC ATT TAT GAT GAG GAT GAA

    ATT CAG AGC CCC CGC AGC TTT

    CAG AAG AAA ACA CGA CAC TAT

    TTT ATT GCT GCA GTG GAG AGG 3'
```

und

(c) für ein Protein codiert, das nach Expression Gerinnungsaktivität des menschlichen Faktors VIII:C zeigt, und

(2) Gewinnung des Proteins aus der Kultur.

8. Transformierte nicht-menschliche Säugerzelle, die ein für menschlichen Faktor VIII:C codierendes Gen enthält, das ein Polydesoxyribonukleotid mit der Sequenz:

```
5' CGC AGC TTT CAG AAG AAA ACA CGA CAC

    TAT TTT ATT GCT GCA GTG GAG AGG 3'
```

in operabler Verbindung mit einer heterologen regulatorischen Kontrollsequenz in einem Expressionsvektor hierfür umfaßt.

# FIG. I

**A. Amino Acid Sequence of 69 Kd Porcine AKF Fragment**

| | | | | | | | | |
|---|---|---|---|---|---|---|---|---|
| Phe | Gln | Lys | Arg | Thr | Arg | His | Tyr | Phe |
| Ile | Ala | Ala | Val | Glu | Gln | Leu | Trp | Asp |
| Tyr | Gly | Met | Ser | Glu | Ser | Pro | Arg | Ala |
| Leu | Arg | Asn | Arg | Ala | Gln | Asn | Gly | Glu |
| Val | Pro | Arg | Phe | Lys | | | | |

**B. Amino Acid Sequence Encoded by Porcine AHF Exon (34-H1)**

| | | | | | | | | | | |
|---|---|---|---|---|---|---|---|---|---|---|
| Arg | Ser | Phe | Gln | Lys | Arg | Thr | Arg | His | Tyr | Phe |
| | | Ile | Ala | Ala | Val | Glu | Gln | Leu | Trp | Asp |
| | | Tyr | Gly | Met | Ser | Glu | Ser | Pro | Arg | Ala |

**C. Amino Acid Sequence Encoded in Human AHF Exon (25-S1)**

| | | | | | | | | | |
|---|---|---|---|---|---|---|---|---|---|
| Arg | Ser | Phe | Gln | Lys | Lys | Thr | Arg | His | Tyr |
| | Phe | Ile | Ala | Ala | Val | Glu | Arg | | |

EP 0 162 067 B1

# FIG. 2

**A.  Amines Terminus of 166Kd Polypeptide**

X I R R Y Y L G A V E L S W D Y R Q S E L L R E L H V D T R F P A

**B.  Fragment of 166Kd Polypeptide**

X V A K K H P K T W V H Y I S A E E E D W D Y A P A V P S P S D R S/T Y K S L

## FIG. 3

| Amino Acid No. | 8 | 9 | 10 | 11 | 12 | 13 | 14 | 15 | 16 | 17 | 18 | 19 | 20 | 21 | 22 |
|---|---|---|---|---|---|---|---|---|---|---|---|---|---|---|---|
| Amino Acid Sequence | His | Tyr | Phe | Ile | Ala | Ala | Val | Glu | Gln | Leu | Trp | Asp | Tyr | Gly | Met |
| Possible mRNA Sequences | CAU CAC | UAU UAC | UUU UUC | AUU AUC AUA | GCU GCC GCA GCG | GCU GCC GCA GCG | GUU GUC GUA GUG | GAA GAG | CAA CAG | CUU CUC CUA CUG UUA UUG | UGG | GAU GAC | UAU UAC | GGU GGC GGA GBG | AUG |
| Guessed Probe Sequence | GTG | ATG | AAA AAG | TAA | CGA | CGA | CAC | CTT CTC | GTC | GAC | ACC | CTA CTG | ATA | CCG | TAC |
| Actual Probe Sequence | GTG | ATA | AAG | TAA | CGA | CGC | CAC | CTC | GTC | GAG | ACC | CTA | ATG | CCC | TAC |

# FIG. 4

| | | | | | | | | |
|---|---|---|---|---|---|---|---|---|
| AAA | ACA | CGG | GGC | ACC | TGT | TAA | CCT | 27<br>GAA |
| CAA | AGT | AAA | TAG | ACC | TGG | AAG | GAC | 54<br>TCC |
| CTC | CAA | GCT | TCT | GGG | TCC | CCC | GAT | 81<br>GCC |
| CAA | AGA | GTG | GGA | ATC | CCT | AGA | GAA | 108<br>GTC |
| ACC | AAA | AAG | CAA | AGC | TCT | CAG | GAC | 135<br>GAA |
| AGA | CAT | CAT | CAG | TTT | ACC | CCT | GGA | 162<br>CCG |
| TCA | CGA | AAG | CAA | TCA | TTC | AAT | AGC | 189<br>AGC |
| AAA | AAA | TGA | AGG | ACA | AGC | CGA | GAC | 216<br>CCA |
| AAG | AGA | AGC | CGC | CTG | GAC | GAA | GCA | 243<br>GGG |
| AGG | GCC | TGG | AAG | GCT | GTG | CGC | TCC | 270<br>AAA |
| GCC | TCC | GGT | CCT | GCG | ACG | GCA | TCA | 297<br>GAG |
| GGA | CAT | AAG | CCT | TCC | TAC | TTT | TJA* | 324<br>CCC |
| GGA | GGA | AGA | CAA | AAT | GGA | CTA | TGA | 351<br>TGA |
| TAT | CTT | CTA | ACT | GAA | ACG | AAG | GGA | 378<br>GAA |
| GAT | TTT | GAC | ATT | TAC | GGT | GAG | GAT | 405<br>GAA |
| AAT | CAG | GAC | CCT | CGC | AGC | TTT | CAG | 432<br>AAG |
| AGA | ACC | CGA | CAC | TAT | TTC | ATT | GCT | 459<br>GCG |
| GTG | GAG | CAG | CTC | TGG | GAT | TAC | GGG | 486<br>ATG |
| AGC | GAA | TCC | CCC | CGG | GCG | CTA | AGA | 513<br>AAC |
| AGG | TAT | GGC | TAC | GTT | GGC | TAC | TCC | 540<br>TCT |
| GTC | CTA | CCC | TGG | GGA | CCT | TTG | TCT | 567<br>TGA |
| GCA | GGT | GCC | GAA | GCC | ATG | GGA | AAG | 594<br>GCA |
| CAA | GCA | GTC | TGG | GGG | TGG | AGA | GGC | 621<br>CAC |
| AGT | GGG | AGG | ATG | TGC | TTG | TTG | GGG | 648<br>AGC |
| ACA | GCG | TGG | TCG | GGC | AGG | GAA | GAG | 675<br>CAG |
| ACC | GAC | CTG | AGG | AGA | G | | | |

\* J Indicates an ambiguity, in accordance with the Stanford code.

# FIG.5

```
   (250)            9                 18               27
5'     TGG  AAG  GCT  GTG  CGC  TCC  AAA  GCC  TCC  GGT  CCT


       36               45                               63
      GCG  ACG  GCA  TCA  GAG  GGA  CAT  AAG  CCT  TCC  TAC


            72               81               90              99
      TTT  TJA  CCC  GGA  GGA  AGA  CAA  AAT  GGA  CTA  TGA


                 108              117              126
      TGA  TAT  CTT  CTA  ACT  GAA  ACG  AAG  GGA  GAA  GAT


       135              144              153              162
      TTT  GAC  ATT  TAC  GGT  GAG  GAT  GAA  AAT  CAG  GAC


            171              180              189              198
      CCT  CGC  AGC  TTT  CAG  AAG  AGA  ACC  CGA  CAC  TAT
            R    S    F    Q    K    R    T    R    H    Y


                 207              216              225
      TTC  ATT  GCT  GCG  GTG  GAG  CAG  CTC  [TGG  GAT  TAC
      F    I    A    A    V    E    Q    L    W    D    Y


       234              243              252              261
      GGG  ATG] AGC  GAA  TCC  CCC  CGG  GCG  CTA  AGA
      G    M    S    E    S    P    R    A    L    R


            267
      AAC  AGG  TAT  GGC  TAC  GTT  GGC  TAC  TCC  TCT
      N    R


      GTC  CTA  CCC  TGG  GGA  CCT  TTG  TCT  TGA  GCA


      GGT  GCC  GAA  GCC  ATG  GGA  AAG  GCA  CAA  GCA


      GTC  TGG  GGG  TGG  AGA  (3')
```

# FIG.6

HUMAN SEQ. DATA  (Complement of 15 mer desired sequence)

```
5'    GAC   ATT   TAT   GAT   GAG   GAT   GAA


      ATT   CAG   AGC   CCC   CGC   AGC   TTT


      CAG   AAG   AAA   ACA   CGA   CAC   TAT


      TTT   ATT   GCT   GCA   GTG   GAG   AGG
```

# FIG. 7

5' GAATTCCGCAGTGGGTAAGTTCCTTAAATGCTCTGGAAAGAAATTCGGACTTTTCATTAAATCAGAAATT

TTACTTTTTTCCCCTCCTGCGAGCTAAAGATATTTTAGAGAAGAATTAACCTTTTGCTTCTCCAGTTGAACATTTCTAGCAATAAGTC

```
MET  Gln  Ile  Glu  Leu  Ser  Thr  Cys  Phe  Phe  Leu  Cys  Leu  Leu  Arg  Phe  Cys  Phe   18
ATG  CAA  ATA  GAG  CTC  TCC  ACC  TGC  TTC  TTT  CTG  TGC ,CTT  TTG  CGA  TTC  TGC  TTT

Ser  Ala  Thr  Arg  Arg  Tyr  Tyr  Leu  Gly  Ala  Val  Glu  Leu  Ser  Trp  Asp  Tyr  MET   36
AGT  GCC  ACC  AGA  AGA  TAC  TAC  CTG  GGT  GCA  GTG  GAA  CTG  TCA  TGG  GAC  TAT  ATC

Gln  Ser  Asp  Leu  Gly  Glu  Leu  Pro  Val  Asp  Ala  Arg  Phe  Pro  Pro  Arg  Val  Pro   54
CAA  AGT  GAT  CTC  GGT  GAG  CTG  CCT  GTG  GAC  GCA  AGA  TTT  CCT  CCT  AGA  GTG  CCA

Lys  Ser  Phe  Pro  Phe  Asn  Thr  Ser  Val  Val  Tyr  Lys  Lys  Thr  Leu  Phe  Val  Glu   72
AAA  TCT  TTT  CCA  TTC  AAC  ACC  TCA  GTC  GTG  TAC  AAA  AAG  ACT  CTG  TTT  GTA  GAA

Phe  Thr  Val  His  Leu  Phe  Asn  Ile  Ala  Lys  Pro  Arg  Pro  Pro  Trp  MET  Gly  Leu   90
TTC  ACG  GTT  CAC  CTT  TTC  AAC  ATC  CCT  AAG  CCA  AGG  CCA  CCC  TGG  ATG  GCT  CTG

Leu  Gly  Pro  Thr  Ile  Gln  Ala  Glu  Val  Tyr  Asp  Thr  Val  Val  Ile  Thr  Leu  Lys  108
CTA  GGT  CCT  ACC  ATC  CAG  GCT  GAG  GTT  TAT  GAT  ACA  GTG  GTC  ATT  ACA  CTT  AAG

Asn  MET  Ala  Ser  His  Pro  Val  Ser  Leu  His  Ala  Val  Gly  Val  Ser  Tyr  Trp  Lys  126
AAC  ATG  GCT  TCC  CAT  CCT  GTC  ACT  CTT  CAT  GCT  GTT  GGT  GTA  TCC  TAC  TGG  AAA

Ala  Ser  Glu  Gly  Ala  Glu  Tyr  Asp  Asp  Gln  Thr  Ser  Gln  Arg  Glu  Lys  Glu  Asp  144
GCT  TCT  GAG  GGA  GCT  GAA  TAT  GAT  GAT  CAG  ACC  ACT  CAA  AGG  GAG  AAA  GAA  GAT

Asp  Lys  Val  Phe  Pro  Gly  Gly  Ser  His  Thr  Tyr  Val  Trp  Gln  Val  Leu  Lys  Glu  162
GAT  AAA  GTC  TTC  CCT  GGT  GGA  AGC  CAT  ACA  TAT  GTC  TGG  CAG  GTC  CTG  AAA  GAG

Asn  Gly  Pro  MET  Ala  Ser  Asp  Pro  Leu  Cys  Leu  Thr  Tyr  Ser  Tyr  Leu  Ser  His  180
AAT  GGT  CCA  ATG  GCC  TCT  GAC  CCA  CTG  TGC  CTT  ACC  TAC  TCA  TAT  CTT  TCT  CAT

Val  Asp  Leu  Val  Lys  Asp  Leu  Asn  Ser  Gly  Leu  Ile  Gly  Ala  Leu  Leu  Val  Cys  198
GTC  GAC  CTG  GTA  AAA  GAC  TTG  AAT  TCA  CGC  CTC  ATT  GGA  GCC  CTA  CTA  GTA  TGT

Arg  Glu  Gly  Ser  Leu  Ala  Lys  Glu  Lys  Thr  Gln  Thr  Leu  His  Lys  Phe  Ile  Leu  216
AGA  GAA  GGG  AGT  CTG  GCC  AAG  GAA  AAG  ACA  CAG  ACC  TTG  CAC  AAA  TTT  ATA  CTA

Leu  Phe  Ala  Val  Phe  Asp  Glu  Gly  Lys  Ser  Trp  His  Ser  Glu  Thr  Lys  Asn  Ser  234
CTT  TTT  GCT  GTA  TTT  GAT  GAA  GGG  AAA  AGT  TGG  CAC  TCA  GAA  ACA  AAG  AAC  TCC

Leu  MET  Gln  Asp  Arg  Asp  Ala  Ala  Ser  Ala  Arg  Ala  Trp  Pro  Lys  MET  His  Thr  252
TTG  ATG  CAG  GAT  AGG  GAT  GCT  GCA  TCT  CCT  CGG  GCC  TGG  CCT  AAA  ATG  CAC  ACA

Val  Asn  Gly  Tyr  Val  Asn  Arg  Ser  Leu  Pro  Gly  Leu  Ile  Gly  Cys  His  Arg  Lys  270
GTC  AAT  GGT  TAT  GTA  AAC  AGG  TCT  CTG  CCA  GGT  CTG  ATT  GGA  TGC  CAC  AGG  AAA

Ser  Val  Tyr  Trp  His  Val  Ile  Gly  MET  Gly  Thr  Thr  Pro  Glu  Val  His  Ser  Ile  288
TCA  GTC  TAT  TGG  CAT  GTG  ATT  GGA  ATG  GGC  ACC  ACT  CCT  GAA  GTG  CAC  TCA  ATA

Phe  Lue  Glu  Gly  His  Thr  Phe  Leu  Val  Arg  Asn  His  Arg  Gln  Ala  Ser  Leu  Glu  306
TTC  CTC  GAA  GGT  CAC  ACA  TTT  CTT  CTG  AGG  AAC  CAT  CGC  CAC  CCG  TCC  TTG  GAA
```

35

2

```
Ile Ser Pro Ile Thr Phe Leu Thr Ala Gln Thr Leu Leu MET Asp Leu Gly Gln   324
ATC TCG CCA ATA ACT TTC CTT ACT GCT CAA ACA CTC TTG ATG GAC CTT GGA CAG

Phe Leu Leu Phe Cys His Ile Ser Ser His Gln His Asp Gly MET Glu Ala Tyr   342
TTT CTA CTG TTT TCT CAT ATC TCT TCC CAC CAA CAT GAT GGC ATG GAA GCT TAT

Val Lys Val Asp Ser Cys Pro Glu Glu Pro Gln Leu Arg MET Lys Asn Asn Glu   360
GTC AAA GTA GAC AGC TGT CCA GAG GAA CCC CAA CTA CGA ATG AAA AAT AAT GAA

Glu Ala Glu Asp Tyr Asp Asp Asp Leu Thr Asp Ser Glu MET Asp Val Val Arg   378
GAA GCG GAA GAC TAT GAT GAT GAT CTT ACT GAT TCT GAA ATG GAT GTG GTC AGG

Phe Asp Asp Asp Asn Ser Pro Ser Phe Ile Gln Ile Arg Ser Val Ala Lys Lys   396
TTT GAT GAT GAC AAC TCT CCT TCC TTT ATC CAA ATT CGC TCA GTT GCC AAG AAG

His Pro Lys Thr Trp Val His Tyr Ile Ala Ala Glu Glu Glu Asp Trp Asp Tyr   414
CAT CCT AAA ACT TGG GTA CAT TAC ATT GCT GCT GAA GAG GAG GAC TGG GAC TAT

Ala Pro Leu Val Leu Ala Pro Asp Asp Arg Ser Tyr Lys Ser Gln Tyr Leu Asn   432
GCT CCC TTA GTC CTC GCC CCC GAT GAC AGA AGT TAT AAA AGT CAA TAT TTG AAC

Asn Gly Pro Gln Arg Ile Gly Arg Lys Tyr Lys Lys Val Arg Phe MET Ala Tyr   450
AAT GGC CCT CAG CGG ATT GGT ACG AAG TAC AAA AAA CTC CGA TTT ATG GCA TAC

Thr Asp Glu Thr Phe Lys Thr Arg Glu Ala Ile Gln His Glu Ser Gly Ile Leu   468
ACA GAT GAA ACC TTT AAG ACT CGT GAA GCT ATT CAG CAT GAA TCA GGA ATC TTG

Gly Pro Leu Leu Tyr Gly Glu Val Gly Asp Thr Leu Leu Ile Ile Phe Lys Asn   486
GGA CCT TTA CTT TAT GGC GAA GTT GGA GAC ACA CTG TTG ATT ATA TTT AAG AAT

Gln Ala Ser Arg Pro Tyr Asn Ile Tyr Pro His Gly Ile Thr Asp Val Arg Pro   504
CAA GCA AGC AGA CCA TAT AAC ATC TAC CCT CAC GGA ATC ACT GAT GTC CGT CCT

Leu Tyr Ser Arg Arg Leu Pro Lys Gly Val Lys His Leu Lys Asp Phe Pro Ile   522
TTG TAT TCA AGG AGA TTA CCA AAA GGT GTA AAA CAT TTG AAG GAT TTT CCA ATT

Leu Pro Gly Glu Ile Phe Lys Tyr Lys Trp Thr Val Thr Val Glu Asp Gly Pro   540
CTG CCA GGA GAA ATA TTC AAA TAT AAA TGG ACA GTG ACT GTA GAA GAT GGG CCA

Thr Lys Ser Asp Pro Arg Cys Leu Thr Arg Tyr Tyr Ser Ser Phe Val Asn MET   558
ACT AAA TCA GAT CCT CGG TGC CTG ACC CGC TAT TAC TCT AGT TTC GTT AAT ATG

Glu Arg Asp Leu Ala Ser Gly Leu Ile Gly Pro Leu Leu Ile Cys Tyr Lys Glu   576
GAG AGA GAT CTA GCT TCA GGA CTC ATT GGC CCT CTC CTC ATC TGC TAC AAA GAA

Ser Val Asp Gln Arg Gly Asn Gln Ile MET Ser Asp Lys Arg Asn Val Ile Leu   594
TCT GTA GAT CAA AGA GGA AAC CAG ATA ATG TCA GAC AAG AGG AAT GTC ATC CTG

Phe Ser Val Phe Asp Glu Asn Arg Ser Trp Tyr Leu Thr Glu Asn Ile Gln Arg   612
TTT TCT GTA TTT GAT GAG AAC CGA AGC TGG TAC CTC ACA GAG AAT ATA CAA CGC

Phe Leu Pro Asn Pro Ala Gly Val Gln Leu Glu Asp Pro Glu Phe Gln Ala Ser   630
TTT CTC CCC AAT CCA GCT GGA GTG CAG CTT GAG GAT CCA GAG TTC CAA GCC TCC

Asn Ile MET His Ser Ile Asn Gly Tyr Val Phe Asp Ser Leu Gln Leu Ser Val   648
AAC ATC ATG CAC AGC ATC AAT GGC TAT GTT TTT GAT AGT TTG CAG TTG TCA GTT
```

36

**3**

| Cys | Leu | His | Glu | Val | Ala | Tyr | Trp | Tyr | Ile | Leu | Ser | Ile | Gly | Ala | Gln | Thr | Asp | 666 |
| TGT | TTG | CAT | GAG | GTG | GCA | TAC | TGG | TAC | ATT | CTA | ACC | ATT | GGA | GCA | CAG | ACT | GAC | |

| Phe | Leu | Ser | Val | Phe | Phe | Ser | Gly | Tyr | Thr | Phe | Lys | His | Lys | MET | Val | Tyr | Glu | 684 |
| TTC | CTT | TCT | GTC | TTC | TTC | TCT | GGA | TAT | ACC | TTC | AAA | CAC | AAA | ATG | GTC | TAT | GAA | |

| Asp | Thr | Leu | Thr | Leu | Phe | Pro | Phe | Ser | Gly | Glu | Thr | Val | Phe | MET | Ser | MET | Glu | 702 |
| GAC | ACA | CTC | ACC | CTA | TTC | CCA | TTC | TCA | GGA | GAA | ACT | GTC | TTC | ATG | TCG | ATG | GAA | |

| Asn | Pro | Gly | Leu | Trp | Ile | Leu | Gly | Cys | His | Asn | Ser | Asp | Phe | Arg | Asn | Arg | Gly | 720 |
| AAC | CCA | GGT | CTA | TGG | ATT | CTG | GGG | TGC | CAC | AAC | TCA | GAC | TTT | CGG | AAC | AGA | CGC | |

| MET | Thr | Ala | Leu | Leu | Lys | Val | Ser | Ser | Cys | Asp | Lys | Asn | Thr | Gly | Asp | Tyr | Tyr | 738 |
| ATG | ACC | GCC | TTA | CTG | AAC | GTT | TCT | AGT | TGT | GAC | AAG | AAC | ACT | GGT | GAT | TAT | TAC | |

| Glu | Asp | Ser | Tyr | Glu | Asp | Ile | Ser | Ala | Tyr | Leu | Leu | Ser | Lys | Asn | Asn | Ala | Ile | 756 |
| GAG | GAC | AGT | TAT | GAA | GAT | ATT | TCA | GCA | TAC | TTG | CTG | AGT | AAA | AAC | AAT | GCC | ATT | |

| Glu | Pro | Arg | Ser | Phe | Ser | Gln | Asn | Ser | Arg | His | Pro | Ser | Thr | Arg | Gln | Lys | Gln | 774 |
| GAA | CCA | AGA | ACC | TTC | TCC | CAG | AAT | TCA | AGA | CAC | CCT | AGC | ACT | AGG | CAA | AAG | CAA | |

| Phe | Asn | Ala | Thr | Thr | Ile | Pro | Glu | Asn | Asp | Ile | Glu | Lys | Thr | Asp | Pro | Trp | Phe | 792 |
| TTT | AAT | GCC | ACC | ACA | ATT | CCA | GAA | AAT | GAC | ATA | CAG | AAG | ACT | GAC | CCT | TGG | TTT | |

| Ala | His | Arg | Thr | Pro | MET | Pro | Lys | Ile | Gln | Asn | Val | Ser | Ser | Ser | Asp | Leu | Leu | 810 |
| GCA | CAC | AGA | ACA | CCT | ATG | CCT | AAA | ATA | CAA | AAT | GTC | TCC | TCT | AGT | GAT | TTG | TTG | |

| MET | Leu | Leu | Arg | Gln | Ser | Pro | Thr | Pro | His | Gly | Leu | Ser | Leu | Ser | Asp | Leu | Gln | 828 |
| ATG | CTC | TTG | CGA | CAG | AGT | CCT | ACT | CCA | CAT | GGG | CTA | TCC | TTA | TCT | GAT | CTC | CAA | |

| Glu | Ala | Lys | Tyr | Glu | Thr | Phe | Ser | Asp | Asp | Pro | Ser | Pro | Gly | Ala | Ile | Asp | Ser | 846 |
| GAA | GCC | AAA | TAT | GAG | ACT | TTT | TCT | GAT | GAT | CCA | TCA | CCT | GGA | GCA | ATA | CAC | AGT | |

| Asn | Asn | Ser | Leu | Ser | Glu | MET | Thr | His | Phe | Arg | Pro | Gln | Leu | His | His | Ser | Gly | 864 |
| AAT | AAC | AGC | CTG | TCT | GAA | ATG | ACA | CAC | TTC | AGG | CCA | CAG | CTC | CAT | CAC | AGT | CGG | |

| Asp | MET | Val | Phe | Thr | Pro | Glu | Ser | Gly | Leu | Gln | Leu | Arg | Leu | Asn | Glu | Lys | Leu | 882 |
| GAC | ATG | GTA | TTT | ACC | CCT | GAG | TCA | GGC | CTC | CAA | TTA | AGA | TTA | AAT | GAG | AAA | CTG | |

| Gly | Thr | Thr | Ala | Ala | Thr | Glu | Leu | Lys | Lys | Leu | Asp | Phe | Lys | Val | Ser | Ser | Thr | 900 |
| GGG | ACA | ACT | GCA | GCA | ACA | GAG | TTG | AAG | AAA | CTT | GAT | TTC | AAA | GTT | TCT | AGT | ACA | |

| Ser | Asn | Asn | Leu | Ile | Ser | Thr | Ile | Pro | Ser | Asp | Asn | Leu | Ala | Ala | Gly | Thr | Asp | 918 |
| TCA | AAT | AAT | CTG | ATT | TCA | ACA | ATT | CCA | TCA | GAC | AAT | TTG | GCA | GCA | GGT | ACT | GAT | |

| Asn | Thr | Ser | Ser | Leu | Gly | Pro | Pro | Ser | MET | Pro | Val | His | Tyr | Asp | Ser | Gln | Leu | 936 |
| AAT | ACA | AGT | TCC | TTA | GGA | CCC | CCA | AGT | ATG | CCA | GTT | CAT | TAT | GAT | AGT | CAA | TTA | |

| Asp | Thr | Thr | Leu | Phe | Gly | Lys | Lys | Ser | Ser | Pro | Leu | Thr | Glu | Ser | Gly | Gly | Pro | 954 |
| GAT | ACC | ACT | CTA | TTT | GGC | AAA | AAG | TCA | TCT | CCC | CTT | ACT | GAG | TCT | GGT | GGA | CCT | |

| Leu | Ser | Leu | Ser | Glu | Glu | Asn | Asn | Asp | Ser | Lys | Leu | Leu | Glu | Ser | Gly | Leu | MET | 972 |
| CTG | AGC | TTG | AGT | GAA | GAA | AAT | AAT | GAT | TCA | AAG | TTG | TTA | GAA | TCA | GGT | TTA | ATG | |

| Asn | Ser | Gln | Glu | Ser | Ser | Trp | Gly | Lys | Asn | Val | Ser | Ser | Thr | Glu | Ser | Gly | Arg | 990 |
| AAT | ACC | CAA | GAA | AGT | TCA | TGG | GGA | AAA | AAT | GTA | TCC | TCA | ACA | GAG | AGT | GGT | ACC | |

37

4

```
Leu  Phe  Lys  Gly  Lys  Arg  Ala  His  Gly  Pro  Ala  Leu  Leu  Thr  Lys  Asp  Asn  Ala 1,008
TTA  TTT  AAA  GGG  AAA  AGA  GCT  CAT  GGA  CCT  GCT  TTG  TTG  ACT  AAA  GAT  AAT  GCC

Leu  Phe  Lys  Val  Ser  Ile  Ser  Leu  Leu  Lys  Thr  Asn  Lys  Thr  Ser  Asn  Asn  Ser 1,026
TTA  TTC  AAA  GTT  AGC  ATC  TCT  TTG  TTA  AAG  ACA  AAC  AAA  ACT  TCC  AAT  AAT  TCA

Ala  Thr  Asn  Arg  Lys  Thr  His  Ile  Asp  Gly  Pro  Ser  Leu  Leu  Ile  Glu  Asn  Ser 1,044
CCA  ACT  AAT  ACA  AAG  ACT  CAC  ATT  GAT  CGC  CCA  TCA  TTA  TTA  ATT  GAG  AAT  AGT

Pro  Ser  Val  Trp  Gln  Asn  Ile  Leu  Glu  Ser  Asp  Thr  Glu  Phe  Lys  Lys  Val  Thr 1,062
CCA  TCA  GTC  TGG  CAA  AAT  ATA  TTA  GAA  AGT  GAC  ACT  GAG  TTT  AAA  AAA  GTG  ACA

Pro  Leu  Ile  His  Asp  Arg  MET  Leu  MET  Asp  Lys  Asn  Ala  Thr  Ala  Leu  Arg  Leu 1,080
CCT  TTG  ATT  CAT  GAC  AGA  ATG  CTT  ATG  GAC  AAA  AAT  GCT  ACA  GCT  TTC  AGG  CTA

Asn  His  MET  Ser  Asn  Lys  Thr  Thr  Ser  Ser  Lys  Asn  MET  Glu  MET  Val  Gln  Gln 1,098
AAT  CAT  ATG  TCA  AAT  AAA  ACT  ACT  TCA  TCA  AAA  ACC  ATG  GAA  ATG  GTC  CAA  CAG

Lys  Lys  Glu  Gly  Pro  Ile  Pro  Pro  Asp  Ala  Gln  Asn  Pro  Asp  MET  Ser  Phe  Phe 1,116
AAA  AAA  GAG  GGC  CCC  ATT  CCA  CCA  GAT  GCA  CAA  AAT  CCA  GAT  ATG  TCG  TTC  TTT

Lys  MET  Leu  Phe  Leu  Pro  Glu  Ser  Ala  Arg  Trp  Ile  Gln  Arg  Thr  His  Gly  Lys 1,134
AAG  ATG  CTA  TTC  TTG  CCA  GAA  TCA  GCA  AGG  TGG  ATA  CAA  ACG  ACT  CAT  GGA  AAG

Asn  Ser  Leu  Asn  Ser  Gly  Gln  Gly  Pro  Ser  Pro  Lys  Gln  Leu  Val  Ser  Leu  Gly 1,152
AAC  TCT  CTG  AAC  TCT  GGG  CAA  GGC  CCC  AGT  CCA  AAG  CAA  TTA  GTA  TCC  TTA  GCA

Pro  Glu  Lys  Ser  Val  Glu  Gly  Gln  Asn  Phe  Leu  Ser  Glu  Lys  Asn  Lys  Val  Val 1,170
CCA  GAA  AAA  TCT  GTG  GAA  GGT  CAG  AAT  TTC  TTG  TCT  GAG  AAA  AAC  AAA  GTG  GTA

Val  Gly  Lys  Gly  Glu  Phe  Thr  Lys  Asp  Val  Gly  Leu  Lys  Glu  MET  Val  Phe  Pro 1,188
GTA  GGA  AAG  GGT  GAA  TTT  ACA  AAG  GAC  GTA  GGA  CTC  AAA  GAG  ATG  GTT  TTT  CCA

Ser  Ser  Arg  Asn  Leu  Phe  Leu  Thr  Asn  Leu  Asp  Asn  Leu  His  Glu  Asn  Asn  Thr 1,206
AGC  AGC  AGA  AAC  CTA  TTT  CTT  ACT  AAC  TTG  GAT  AAT  TTA  CAT  GAA  AAT  AAT  ACA

His  Asn  Gln  Glu  Lys  Lys  Ile  Gln  Glu  Glu  Ile  Glu  Lys  Lys  Glu  Thr  Leu  Ile 1,224
CAC  AAT  CAA  CAA  AAA  AAA  ATT  CAG  GAA  GAA  ATA  GAA  AAG  AAG  GAA  ACA  TTA  ATC

Gln  Glu  Asn  Val  Val  Leu  Pro  Gln  Ile  His  Thr  Val  Thr  Gly  Thr  Lys  Asn  Phe 1,242
CAA  GAG  AAT  GTA  GTT  TTG  CCT  CAG  ATA  CAT  ACA  GTG  ACT  GGC  ACT  AAG  AAT  TTC

MET  Lys  Asn  Leu  Phe  Leu  Leu  Ser  Thr  Arg  Gln  Asn  Val  Glu  Gly  Ser  Tyr  Glu 1,260
ATG  AAC  AAC  CTT  TTC  TAA  CTG  AGC  ACT  AGG  CAA  AAT  GTA  GAA  GGT  TCA  TAT  GAG

Gly  Ala  Tyr  Ala  Pro  Val  Leu  Gln  Asp  Phe  Arg  Ser  Leu  Asn  Asp  Ser  Thr  Asn 1,278
GGG  GCA  TAT  GCT  CCA  GTA  CTT  CAA  GAT  TTT  AGG  TCA  TTA  AAT  CAT  TCA  ACA  AAT

Arg  Thr  Lys  Lys  His  Thr  Ala  His  Phe  Ser  Lys  Lys  Gly  Glu  Glu  Glu  Asn  Leu 1,296
AGA  ACA  AAC  AAA  CAC  ACA  GCT  CAT  TTC  TCA  AAA  AAA  GGC  GAG  GAA  CAA  AAC  TTG

Glu  Gly  Leu  Gly  Asn  Gln  Thr  Lys  Gln  Ile  Val  Glu  Lys  Tyr  Ala  Cys  Thr  Thr 1,314
GAA  GGC  TTG  GGA  AAT  CAA  ACC  AAG  CAA  ATT  GTA  GAG  AAA  TAT  GCA  TGC  ACC  ACA

Arg  Ile  Ser  Pro  Asn  Thr  Ser  Gln  Gln  Asn  Phe  Val  Thr  Gln  Arg  Ser  Lys  Arg 1,332
AGG  ATA  TCT  CCT  AAT  ACA  AGC  CAG  CAG  AAT  TTT  GTC  ACG  CAA  CGT  AGT  AAG  AGA
```

38

**5**

```
Ala Leu Lys Gln Phe Arg Leu Pro Leu Glu Glu Thr Glu Leu Glu Lys Arg Ile  1,350
GCT TTG AAA CAA TTC AGA CTC CCA CTA GAA GAA ACA GAA CTT GAA AAA AGC ATA

Ile Val Asp Asp Thr Ser Thr Gln Trp Ser Lys Asn MET Lys His Leu Thr Pro  1,368
ATT GTG GAT GAC ACC TCA ACC CAC TGG TCC AAA AAC ATG AAA CAT TTG ACC CCG

Ser Thr Leu Thr Gln Ile Asp Tyr Asn Glu Lys Glu Lys Gly Ala Ile Thr Gln  1,386
AGC ACC CTC ACA CAG ATA GAC TAC AAT GAG AAG GAG AAA GGG GCC ATT ACT CAC

Ser Pro Leu Ser Asp Cys Leu Thr Arg Ser His Ser Ile Pro Gln Ala Asn Arg  1,404
TCT CCC TTA TCA GAT TGC CTT ACG AGG ACT CAT AGC ATC CCT CAA GCA AAT AGA

Ser Pro Leu Pro Ile Ala Lys Val Ser Ser Phe Pro Ser Ile Arg Pro Ile Tyr  1,422
TCT CCA TTA CCC ATT GCA AAG GTA TCA TCA TTT CCA TCT ATT AGA CCT ATA TAT

Leu Thr Arg Val Leu Phe Gln Asp Asn Ser Ser His Leu Pro Ala Ala Ser Tyr  1,440
CTG ACC AGG GTC CTA TTC CAA GAC AAC TCT TCT CAT CTT CCA GCA GCA TCT TAT

Arg Lys Lys Asp Ser Gly Val Gln Glu Ser Ser His Phe Leu Gln Gly Ala Lys  1,458
ACA AAG AAA GAT TCT GGG GTC CAA GAA AGC ACT CAT TTC TTA CAA GGA GCC AAA

Lys Asn Asn Leu Ser Leu Ala Ile Leu Thr Leu Glu MET Thr Gly Asp Gln Arg  1,476
AAA AAT AAC CTT TCT TTA GCC ATT CTA ACC TTG GAG ATG ACT GGT GAT CAA AGA

Glu Val Gly Ser Leu Gly Thr Ser Ala Thr Asn Ser Val Thr Tyr Lys Lys Val  1,494
GAG GTT GGC TCC CTG GGG ACA AGT GCC ACA AAT TCA GTC ACA TAC AAG AAA GTT

Glu Asn Thr Val Leu Pro Lys Pro Asp Leu Pro Lys Thr Ser Gly Lys Val Glu  1,512
GAG AAC ACT GTT CTC CCG AAA CCA GAC TTG CCC AAA ACA TCT GGC AAA GTT GAA

Leu Leu Pro Lys Val His Ile Tyr Gln Lys Asp Leu Phe Pro Thr Glu Thr Ser  1,530
TTG CTT CCA AAA GTT CAC ATT TAT CAG AAG GAC CTA TTC CCT ACG GAA ACT AGC

Asn Gly Ser Pro Gly His Leu Asp Leu Val Glu Gly Ser Leu Leu Gln Gly Thr  1,548
AAT GGG TCT CCT GGC CAT CTG GAT CTC GTG GAA GGG AGC CTT CTT CAG GGA ACA

Glu Gly Ala Ile Lys Trp Asn Glu Ala Asn Arg Pro Gly Lys Val Pro Phe Leu  1,566
GAG GGA GCG ATT AAC TGG AAT GAA CCA AAC AGA CCT CGA AAA GTT CCC TTT CTG

Arg Val Ala Thr Glu Ser Ser Ala Lys Thr Pro Ser Lys Leu Leu Asp Pro Leu  1,584
AGA GTA GCA ACA GAA AGC TCT GCA AAG ACT CCC TCC AAC CTA TTG GAT CCT CTT

Ala Trp Asp Asn His Tyr Gly Thr Gln Ile Pro Lys Glu Glu Trp Lys Ser Gln  1,602
GCT TGG GAT AAC CAC TAT GGT ACT CAG ATA CCA AAA GAA GAG TGG AAA TCC CAA

Glu Lys Ser Pro Glu Lys Thr Ala Phe Lys Lys Lys Asp Thr Ile Leu Ser Leu  1,520
GAG AAG TCA CCA GAA AAA ACA GCT TTT AAG AAA AAG GAT ACC ATT TTG TCC CTG

Asn Ala Cys Glu Ser Asn His Ala Ile Ala Ala Ile Asn Glu Gly Gln Asn Lys  1,638
AAC GCT TGT GAA AGC AAT CAT GCA ATA GCA GCA ATA AAT GAG GGA CAA AAT AAG

Pro Glu Ile Glu Val Thr Trp Ala Lys Gln Gly Arg Thr Glu Arg Leu Cys Ser  1,656
CCC GAA ATA GAA GTC ACC TGG GCA AAG CAA GGT ACG ACT GAA AGG CTC TGC TCT

Gln Asn Pro Pro Val Leu Lys Arg His Gln Arg Glu Ile Thr Arg Thr Thr Leu  1,674
CAA AAC CCA CCA GTC TTC AAA CGC CAT CAA CGC GAA ATA ACT CGT ACT ACT CTT
```

**6**

| | | | | | | | | | | | | | | | | | | |
|---|---|---|---|---|---|---|---|---|---|---|---|---|---|---|---|---|---|---|
| Gln | Ser | Asp | Gln | Glu | Glu | Ile | Asp | Tyr | Asp | Asp | Thr | Ile | Ser | Val | Glu | MET | Lys | 1,692 |
| CAG | TCA | GAT | CAA | GAG | GAA | ATT | GAC | TAT | GAT | CAT | ACC | ATA | TCA | GTT | GAA | ATG | AAG | |

| | | | | | | | | | | | | | | | | | | |
|---|---|---|---|---|---|---|---|---|---|---|---|---|---|---|---|---|---|---|
| Lys | Glu | Asp | Phe | Asp | Ile | Tyr | Asp | Glu | Asp | Glu | Asn | Gln | Ser | Pro | Arg | Ser | Phe | 1,710 |
| AAG | GAA | GAT | TTT | GAC | ATT | TAT | GAT | GAG | GAT | GAA | AAT | CAG | AGC | CCC | CGC | AGC | TTT | |

| | | | | | | | | | | | | | | | | | | |
|---|---|---|---|---|---|---|---|---|---|---|---|---|---|---|---|---|---|---|
| Gln | Lys | Lys | Thr | Arg | His | Tyr | Phe | Ile | Ala | Ala | Val | Glu | Arg | Leu | Trp | Asp | Tyr | 1,728 |
| CAA | AAG | AAA | ACA | CGA | CAC | TAT | TTT | ATT | GCT | GCA | GTG | GAG | AGG | CTC | TGG | GAT | TAT | |

| | | | | | | | | | | | | | | | | | | |
|---|---|---|---|---|---|---|---|---|---|---|---|---|---|---|---|---|---|---|
| Gly | MET | Ser | Ser | Ser | Pro | His | Val | Leu | Arg | Asn | Arg | Ala | Gln | Ser | Gly | Ser | Val | 1,746 |
| GGG | ATG | AGT | AGC | TCC | CCA | CAT | GTT | CTA | AGA | AAC | AGG | GCT | CAG | AGT | GGC | AGT | GTC | |

| | | | | | | | | | | | | | | | | | | |
|---|---|---|---|---|---|---|---|---|---|---|---|---|---|---|---|---|---|---|
| Pro | Gln | Phe | Lys | Lys | Val | Val | Phe | Gln | Glu | Phe | Thr | Asp | Gly | Ser | Phe | Thr | Gln | 1,764 |
| CCT | CAG | TTC | AAG | AAA | GTT | GTT | TTC | CAG | GAA | TTT | ACT | GAT | GGC | TCC | TTT | ACT | CAG | |

| | | | | | | | | | | | | | | | | | | |
|---|---|---|---|---|---|---|---|---|---|---|---|---|---|---|---|---|---|---|
| Pro | Leu | Tyr | Arg | Gly | Glu | Leu | Asn | Glu | His | Leu | Gly | Leu | Leu | Gly | Pro | Tyr | Ile | 1,782 |
| CCC | TTA | TAC | CGT | GGA | GAA | CTA | AAT | GAA | CAT | TTG | GGA | CTC | CTG | GGG | CCA | TAT | ATA | |

| | | | | | | | | | | | | | | | | | | |
|---|---|---|---|---|---|---|---|---|---|---|---|---|---|---|---|---|---|---|
| Arg | Ala | Glu | Val | Glu | Asp | Asn | Ile | MET | Val | Thr | Phe | Arg | Asn | Gln | Ala | Ser | Arg | 1,800 |
| AGA | GCA | GAA | GTT | GAA | GAT | AAT | ATC | ATG | GTA | ACT | TTC | AGA | AAT | CAG | GCC | TCT | CGT | |

| | | | | | | | | | | | | | | | | | | |
|---|---|---|---|---|---|---|---|---|---|---|---|---|---|---|---|---|---|---|
| Pro | Tyr | Ser | Phe | Tyr | Ser | Ser | Leu | Ile | Ser | Tyr | Glu | Glu | Asp | Gln | Arg | Gln | Gly | 1,818 |
| CCC | TAT | TCC | TTC | TAT | TCT | AGC | CTT | ATT | TCT | TAT | GAG | GAA | GAT | CAG | AGG | CAA | GGA | |

| | | | | | | | | | | | | | | | | | | |
|---|---|---|---|---|---|---|---|---|---|---|---|---|---|---|---|---|---|---|
| Ala | Glu | Pro | Arg | Lys | Asn | Phe | Val | Lys | Pro | Asn | Glu | Thr | Lys | Thr | Tyr | Phe | Trp | 1,836 |
| GCA | GAA | CCT | AGA | AAA | AAC | TTT | GTC | AAG | CCT | AAT | GAA | ACC | AAA | ACT | TAC | TTT | TGG | |

| | | | | | | | | | | | | | | | | | | |
|---|---|---|---|---|---|---|---|---|---|---|---|---|---|---|---|---|---|---|
| Lys | Val | Gln | His | His | MET | Ala | Pro | Thr | Lys | Asp | Glu | Phe | Asp | cys | Lys | Ala | Trp | 1,854 |
| AAA | GTG | CAA | CAT | CAT | ATG | GCA | CCC | ACT | AAA | GAT | GAG | TTT | GAC | TGC | AAA | GCC | TGG | |

| | | | | | | | | | | | | | | | | | | |
|---|---|---|---|---|---|---|---|---|---|---|---|---|---|---|---|---|---|---|
| Ala | Tyr | Phe | Ser | Asp | Val | Asp | Leu | Glu | Lys | Asp | Val | His | Ser | Gly | Leu | Ile | Gly | 1,872 |
| GCT | TAT | TTC | TCT | GAT | GTT | GAC | CTG | GAA | AAA | GAT | GTG | CAC | TCA | GGC | CTG | ATT | GGA | |

| | | | | | | | | | | | | | | | | | | |
|---|---|---|---|---|---|---|---|---|---|---|---|---|---|---|---|---|---|---|
| Pro | Leu | Leu | Val | Cys | His | Thr | Asn | Thr | Leu | Asn | Pro | Ala | His | Gly | Arg | Gln | Val | 1,890 |
| CCC | CTT | CTG | CTC | TGC | CAC | ACT | AAC | ACA | CTG | AAC | CCT | GCT | CAT | CCG | AGA | CAA | GTG | |

| | | | | | | | | | | | | | | | | | | |
|---|---|---|---|---|---|---|---|---|---|---|---|---|---|---|---|---|---|---|
| Thr | Val | Gln | Glu | Phe | Ala | Leu | Phe | Phe | Thr | Ile | Phe | Asp | Glu | Thr | Lys | Ser | Trp | 1,908 |
| ACA | GTA | CAG | GAA | TTT | GCT | CTG | TTT | TTC | ACC | ATC | TTT | GAT | GAG | ACC | AAA | AGC | TGG | |

| | | | | | | | | | | | | | | | | | | |
|---|---|---|---|---|---|---|---|---|---|---|---|---|---|---|---|---|---|---|
| Thy | Phe | Thr | Glu | Asn | MET | Glu | Arg | Asn | Cys | Arg | Ala | Pro | Cys | Asn | Ile | Gln | MET | 1,926 |
| TAC | TTC | ACT | GAA | AAT | ATG | GAA | AGA | AAC | TGC | AGG | GCT | CCC | TGC | AAT | ATC | CAG | ATG | |

| | | | | | | | | | | | | | | | | | | |
|---|---|---|---|---|---|---|---|---|---|---|---|---|---|---|---|---|---|---|
| Glu | Asp | Pro | Thr | Phe | Lys | Glu | Asn | Thr | Arg | Phe | His | Ala | Ile | Asn | Gly | Tyr | Ile | 1,944 |
| GAA | GAT | CCC | ACT | TTT | AAA | GAG | AAT | TAT | CGC | TTC | CAT | GCA | ATC | AAT | GGC | TAC | ATA | |

| | | | | | | | | | | | | | | | | | | |
|---|---|---|---|---|---|---|---|---|---|---|---|---|---|---|---|---|---|---|
| MET | Asp | Thr | Leu | Pro | Gly | Leu | Val | MET | Ala | Gln | Asp | Gln | Arg | Ile | Arg | Trp | Tyr | 1,962 |
| ATG | GAT | ACA | CTA | CCT | GGC | TTA | GTA | ATG | GCT | CAG | GAT | CAA | AGG | ATT | CGA | TGG | TAT | |

| | | | | | | | | | | | | | | | | | | |
|---|---|---|---|---|---|---|---|---|---|---|---|---|---|---|---|---|---|---|
| Leu | Leu | Ser | MET | Gly | Ser | Asn | Glu | Asn | Ile | His | Ser | Ile | His | Phe | Ser | Gly | His | 1,980 |
| CTG | CTC | AGC | ATG | GGC | AGC | AAT | GAA | AAC | ATC | CAT | TCT | ATT | CAT | TTC | AGT | GGA | CAT | |

| | | | | | | | | | | | | | | | | | | |
|---|---|---|---|---|---|---|---|---|---|---|---|---|---|---|---|---|---|---|
| Val | Phe | Thr | Val | Arg | Lys | Lys | Glu | Glu | Tyr | Lys | MET | Ala | Leu | Tyr | Asn | Leu | Tyr | 1,998 |
| GTG | TTC | ACT | GTA | CGA | AAA | AAA | GAG | GAG | TAT | AAA | ATG | GCA | CTG | TAC | AAT | CTC | TAT | |

| | | | | | | | | | | | | | | | | | | |
|---|---|---|---|---|---|---|---|---|---|---|---|---|---|---|---|---|---|---|
| Pro | Gly | Val | Phe | Glu | Thr | Val | Glu | MET | Leu | Pro | Ser | Lys | Ala | Gly | Ile | Trp | Arg | 2,016 |
| CCA | GGT | CTT | TTT | GAG | ACA | GTG | GAA | ATG | TTA | CCA | TCC | AAA | GCT | GGA | ATT | TGG | CGG | |

40

7

| Val | Glu | Cys | Leu | Ile | Gly | Glu | His | Leu | His | Ala | Gly | MET | Ser | Thr | Leu | Phe | Leu | 2,034 |
|-----|-----|-----|-----|-----|-----|-----|-----|-----|-----|-----|-----|-----|-----|-----|-----|-----|-----|-------|
| GTG | GAA | TGC | CTT | ATT | GGC | GAG | CAT | CTA | CAT | GCT | GGG | ATG | AGC | ACA | CTT | TTT | CTG | |

| Val | Tyr | Ser | Asn | Lys | Cys | Gln | Thr | Pro | Leu | Gly | MET | Ala | Ser | Gly | His | Ile | Arg | 2,052 |
|-----|-----|-----|-----|-----|-----|-----|-----|-----|-----|-----|-----|-----|-----|-----|-----|-----|-----|-------|
| GTG | TAC | AGC | AAT | AAG | TGT | CAG | ACT | CCC | CTG | GGA | ATG | GCT | TCT | GGA | CAC | ATT | AGA | |

| Asp | Phe | Gln | Ile | Thr | Ala | Ser | Gly | Gln | Tyr | Gly | Gln | Trp | Ala | Pro | Lys | Leu | Ala | 2,070 |
|-----|-----|-----|-----|-----|-----|-----|-----|-----|-----|-----|-----|-----|-----|-----|-----|-----|-----|-------|
| GAT | TTT | CAG | ATT | ACA | GCT | TCA | GGA | CAA | TAT | GGA | CAG | TGG | GCC | CCA | AAG | CTG | GCC | |

| Arg | Leu | His | Tyr | Ser | Gly | Ser | Ile | Asn | Ala | Trp | Ser | Thr | Lys | Glu | Pro | Phe | Ser | 2,088 |
|-----|-----|-----|-----|-----|-----|-----|-----|-----|-----|-----|-----|-----|-----|-----|-----|-----|-----|-------|
| AGA | CTT | CAT | TAT | TCC | GGA | TCA | ATC | AAT | GCC | TGG | AGC | ACC | AAG | GAG | CCC | TTT | TCT | |

| Trp | Ile | Lys | Val | Asp | Leu | Leu | Ala | Pro | MET | Ile | Ile | His | Gly | Ile | Lys | Thr | Gln | 2,106 |
|-----|-----|-----|-----|-----|-----|-----|-----|-----|-----|-----|-----|-----|-----|-----|-----|-----|-----|-------|
| TGG | ATC | AAG | GTG | GAT | CTG | TTG | GCA | CCA | ATG | ATT | ATT | CAC | GGC | ATC | AAG | ACC | CAG | |

| Gly | Ala | Arg | Gln | Lys | Phe | Ser | Ser | Leu | Tyr | Ile | Ser | Gln | Phe | Ile | Ile | MET | Tyr | 2,124 |
|-----|-----|-----|-----|-----|-----|-----|-----|-----|-----|-----|-----|-----|-----|-----|-----|-----|-----|-------|
| GGT | GCC | CGT | CAG | AAG | TTC | TCC | AGC | CTC | TAC | ATC | TCT | CAG | TTT | ATC | ATC | ATG | TAT | |

| Ser | Leu | Asp | Gly | Lys | Lys | Trp | Gln | Thr | Tyr | Arg | Gly | Asn | Ser | Thr | Gly | Thr | Leu | 2,142 |
|-----|-----|-----|-----|-----|-----|-----|-----|-----|-----|-----|-----|-----|-----|-----|-----|-----|-----|-------|
| AGT | CTT | GAT | GGG | AAG | AAG | TGG | CAG | ACT | TAT | CGA | GGA | AAT | TCC | ACT | GGA | ACC | TTA | |

| MET | Val | Phe | Phe | Gly | Asn | Val | Asp | Ser | Ser | Gly | Ile | Lys | His | Asn | Ile | Phe | Asn | 2,160 |
|-----|-----|-----|-----|-----|-----|-----|-----|-----|-----|-----|-----|-----|-----|-----|-----|-----|-----|-------|
| ATG | GTC | TTC | TTT | GGC | AAT | GTC | GAT | TCA | TCT | CGG | ATA | AAA | CAC | AAT | ATT | TTT | AAC | |

| Pro | Pro | Ile | Ile | Ala | Arg | Tyr | Ile | Arg | Leu | His | Pro | Thr | His | Tyr | Ser | Ile | Arg | 2,178 |
|-----|-----|-----|-----|-----|-----|-----|-----|-----|-----|-----|-----|-----|-----|-----|-----|-----|-----|-------|
| CCT | CCA | ATT | ATT | GCT | CGA | TAC | ATC | CGT | TTG | CAC | CCA | ACT | CAT | TAT | AGC | ATT | CGC | |

| Ser | Thr | Leu | Arg | MET | Glu | Leu | MET | Gly | Cys | Asp | Leu | Asn | Ser | Cys | Ser | MET | Pro | 2,196 |
|-----|-----|-----|-----|-----|-----|-----|-----|-----|-----|-----|-----|-----|-----|-----|-----|-----|-----|-------|
| AGC | ACT | CTT | CGC | ATG | GAG | TTG | ATG | GGC | TGT | GAT | TTA | AAT | AGT | TGC | AGC | ATG | CCA | |

| Leu | Gly | MET | Glu | Ser | Lys | Ala | Ile | Ser | Asp | Ala | Gln | Ile | Thr | Ala | Ser | Ser | Tyr | 2,214 |
|-----|-----|-----|-----|-----|-----|-----|-----|-----|-----|-----|-----|-----|-----|-----|-----|-----|-----|-------|
| TTG | GGA | ATG | GAG | AGT | AAA | GCA | ATA | TCA | GAT | GCA | CAG | ATT | ACT | GCT | TCA | TCC | TAC | |

| Phe | Thr | Asn | MET | Phe | Ala | Thr | Trp | Ser | Pro | Ser | Lys | Ala | Arg | Leu | His | Leu | Gln | 2,232 |
|-----|-----|-----|-----|-----|-----|-----|-----|-----|-----|-----|-----|-----|-----|-----|-----|-----|-----|-------|
| TTT | ACC | AAT | ATG | TTT | GCC | ACC | TGG | TCT | CCT | TCA | AAA | GCT | CGA | CTT | CAC | GTC | CAA | |

| Gly | Arg | Ser | Asn | Ala | Trp | Arg | Pro | Gln | Val | Asn | Asn | Pro | Lys | Glu | Trp | Leu | Gln | 2,250 |
|-----|-----|-----|-----|-----|-----|-----|-----|-----|-----|-----|-----|-----|-----|-----|-----|-----|-----|-------|
| GGG | AGG | AGT | AAT | GCC | TGG | AGA | CCT | CAG | GTG | AAT | AAT | CCA | AAA | GAG | TGG | CTG | CAA | |

| Val | Asp | Phe | Gln | Lys | Thr | MET | Lys | Val | Thr | Gly | Val | Thr | Thr | Gln | Gly | Val | Lys | 2,268 |
|-----|-----|-----|-----|-----|-----|-----|-----|-----|-----|-----|-----|-----|-----|-----|-----|-----|-----|-------|
| GTG | GAC | TTC | CAG | AAG | ACA | ATG | AAA | GTC | ACA | GGA | GTA | ACT | ACT | CAG | GGA | GTA | AAA | |

| Ser | Leu | Leu | Thr | Ser | MET | Tyr | Val | Lys | Glu | Phe | Leu | Ile | Ser | Ser | Ser | Gln | Asp | 2,286 |
|-----|-----|-----|-----|-----|-----|-----|-----|-----|-----|-----|-----|-----|-----|-----|-----|-----|-----|-------|
| TCT | CTG | CTT | ACC | ACC | ATG | TAT | GTG | AAG | GAG | TTC | CTC | ATC | TCC | ACC | AGT | CAA | GAT | |

| Gly | His | Gln | Trp | Thr | Leu | Phe | Phe | Gln | Asn | Gly | Lys | Val | Lys | Val | Phe | Gln | Gly | 2,304 |
|-----|-----|-----|-----|-----|-----|-----|-----|-----|-----|-----|-----|-----|-----|-----|-----|-----|-----|-------|
| GGC | CAT | CAG | TGG | ACT | CTC | TTT | TTT | CAG | AAT | GGC | AAA | CTA | AAG | CTT | TTT | CAG | GGA | |

| Asn | Gln | Asp | Ser | Phe | Thr | Pro | Val | Val | Asn | Ser | Leu | Asp | Pro | Pro | Leu | Leu | Thr | 2,322 |
|-----|-----|-----|-----|-----|-----|-----|-----|-----|-----|-----|-----|-----|-----|-----|-----|-----|-----|-------|
| AAT | CAA | GAC | TCC | TTC | ACA | CCT | GTG | GTG | AAC | TCT | CTA | GAC | CCA | CCG | TTA | CTG | ACT | |

| Arg | Tyr | Leu | Arg | Ile | His | Pro | Gln | Ser | Trp | Val | His | Gln | Ile | Ala | Leu | Arg | MET | 2,340 |
|-----|-----|-----|-----|-----|-----|-----|-----|-----|-----|-----|-----|-----|-----|-----|-----|-----|-----|-------|
| CGC | TAC | CTT | CGA | ATT | CAC | CCC | CAG | AGT | TGG | GTG | CAC | CAG | ATT | GCC | CTG | AGG | ATG | |

8

```
                                                                    2,352
Glu  Val  Leu  Gly  Cys  Glu  Ala  Gln  Asp  Leu  Tyr  End
GAG  GTT  CTG  GGC  TGC  GAG  GCA  CAG  GAC  CTC  TAC  TGA  GGGTGGCCACTGCATCCCACCTGCCACTG

CCGTCACCTCTCCCTCCTCAGCTCCAGGGCATGTGTCCCTCCCTGGCTTGCTTCTACCTTTGTGCTAAATCCTAGCAGACACTCCCTTG

AAGCCTCCTGAATTAACTATCATCAGTCCTGCATTTCTTTGGTGGGGGGCCCAGGAGGCTGCATCCATTTTAACTTAACTCTTACCTATT

TTCTGCAGCTGCTCCCAGA
```